# EUROPEAN PATENT SPECIFICATION

(11) **EP 2 217 233 B1**
(45) Date of publication and mention of the grant of the patent: **08.08.2018**
(21) Application number: 08843192.9
(22) Date of filing: 24.10.2008
(51) Int. Cl.: A61K 31/00, A61K 45/06, A61K 31/416, A61K 31/52, A61K 31/522, A61P 43/00, A61P 1/16

(54) **METHODS AND COMPOSITIONS FOR TREATING HEPATIC DISEASES**
VERFAHREN UND ZUSAMMENSETZUNGEN ZUR BEHANDLUNG VON LEBERERKRANKUNGEN
PROCÉDÉS ET COMPOSITIONS POUR LE TRAITEMENT DE MALADIES HÉPATIQUES

(30) Priority: 26.10.2007 US 570
(43) Date of publication of application: 18.08.2010
(73) Proprietor: New York University School of Medicine, New York, NY 10016 (US)
(72) Inventor: CRONSTEIN, Bruce, N., New York NY 10024 (US); PENG, Zhongsheng, New York, NY 10016 (US)
(74) Representative: Büchel, Edwin
(86) International application number: PCT/US2008/012102
(87) International publication number: WO 2009/055021

(56) References cited:
- EP-A- 0 970 696
- WO-A-01/58241
- WO-A-03/022284
- WO-A-2006/115690
- US-A1- 2007 219 221

## Description

### FIELD OF THE INVENTION

The present application discloses methods and compositions for treating hepatic disease. More particularly, the application discloses methods and compositions comprising an A₁ or an A_{2B} adenosine receptor antagonist that act to treat or inhibit the progression of hepatic disease. Such A₁ or A_{2B} adenosine receptor antagonists may be used to treat subjects suffering from, or at risk for developing, diseases or conditions characterized by fat deposition or fibrosis of the liver or by elevated serum lipids, cholesterol or triglycerides.

### BACKGROUND OF THE INVENTION

Adenosine is a nucleoside that occurs naturally in mammals, which acts as a ubiquitous biochemical messenger. The heart, for instance, produces and releases adenosine in order to modulate heart rate and coronary vasodilation. Likewise, adenosine is produced in the kidney to modulate essential physiological responses, including glomerular filtration rate (GFR), electrolyte reabsorption, and renin secretion.

Adenosine is known to bind to and activate seven-transmembrane spanning G-protein coupled receptors, thereby eliciting a variety of physiological responses. There are 4 known subtypes of adenosine receptors (i.e., A₁, A_{2A}, A_{2B}, and A₃), which mediate different, and sometimes opposing, effects. For example, activation of the adenosine A₁ receptor, elicits an increase in renal vascular resistance, which leads to a decrease in glomerular filtration rate (GFR), while activation of the adenosine A_{2A} receptor elicits a decrease in renal vascular resistance. Conversely, blockade of the A₁ adenosine receptor decreases afferent arteriole pressure, leading to an increase in GFR and urine flow, and sodium excretion. Furthermore, A_{2A} adenosine receptors modulate coronary vasodilation, whereas A_{2B} receptors have been implicated in mast cell activation, asthma, vasodilation, regulation of cell growth, intestinal function, and modulation of neurosecretion (*See,* Adenosine A2B Receptors as Therapeutic Targets, Drug Dev Res 45:198; Feoktistov et al., Trends Pharmacol Sci 19:148-153 and Ralevic, V and Burnstock, G. (1998), Pharmacological Reviews, Vol. 50: 413-492), and A₃ adenosine receptors modulate cell proliferation processes. Two receptor subtypes (A₁ and A_{2A}) exhibit affinity for adenosine in the nanomolar range while two other known subtypes A_{2B} and A₃ are low-affinity receptors, with affinity for adenosine in the low-micromolar range. A₁ and A₃ adenosine receptor activation can lead to an inhibition of adenylate cyclase activity, while A_{2A} and A_{2B} activation causes a stimulation of adenylate cyclase.

Diseases that can be prevented and/or treated with A₁ adenosine receptor antagonists include diseases and disorders wherein activation of A₁ adenosine receptors plays a role in pathophysiology. For example, A₁ adenosine receptor antagonists are useful for treating cognitive disorders and dementias such as Alzheimers disease, and for treating stress, depression, cardiac arrhythmia, restoration of cardiac function, congestive heart failure, asthma, and respiratory disorders (e.g., bronchial asthma, allergic lung diseases). They also reduce ischemia-induced injury of the brain, heart and kidney. A₁ adenosine receptor antagonists have pronounced effects on the kidney, and have shown to be potent diuretics and natriuretics with little effect on potassium excretion. Thus, they are also renal protective, useful for the treatment of renal failure, renal dysfunction, nephritis, hypertension, and edema. It has been suggested that A_{2A} antagonists may be beneficial for patients suffering from Morbus Parkinson (Parkinson's disease). Adenosine receptor antagonists may also be valuable for treatment of allergic inflammation and asthma. Available information (for example, Nyce & Metzger "DNA antisense Therapy for Asthma in an Animal Model" Nature (1997) 385: 721-5) indicates that in this pathophysiologic context, A₁ antagonists may block contraction of smooth muscle underlying respiratory epithelia, while A_{2B} or A₃ receptor antagonists may block mast cell degranulation, mitigating the release of histamine and other inflammatory mediators. A_{2B} receptors have been discovered throughout the gastrointestinal tract, especially in the colon and the intestinal epithelia. It has been suggested that A_{2B} receptors mediate cAMP response (Strohmeier et al., J. Bio. Chem. (1995) 270:2387-94).

Hepatic disease can take a wide variety of forms including, but not limited to, necrosis, steatosis, fibrosis, and cholestatis. Other forms of liver disease can result from the ingestion of hepatotoxic medicines such as chemotherapy and cancer drugs, antibiotics, analgesics, antiemetics, and other medications. Also, alcohol and drug abuse are well known causes of liver disease. Typical causes of hepatic disease include, but are not limited to, viral and alcoholic hepatitis, Wilson's disease, hemochromatosis, steatosis, and nonalcoholic steatohepatitis (NASH).

Hepatic fibrosis is a common aspect of many, if not all, hepatic diseases and is defined as the formation of scar tissue in the liver. The scarring develops as the liver attempts to repair cellular damage induced by the ingestion of hepatotoxins, as a consequence of chronic liver inflammation, or as a consequence of physical insult. Hepatic fibrosis may also result as a consequence of surgical intervention and hepatotoxic drug therapy, i.e., liver replacement or repair or chemotherapy. In many cases, hepatic fibrosis produces permanent scarring of the hepatic tissue, a condition commonly referred to as cirrhosis.

Recent studies have disclosed that adenosine plays a role in the development and progression of hepatic fibrosis. Chunn et al. (2006) Am. J. Physiol Lung Cell Mol Physiol, 290(3):L579-87, detected increased hepatic fibrosis in adenosine deaminase (ADA) deficient mice. The mice utilized by Chunn et al. are genetically engineered to possess partial ADA enzyme activity and thereby accumulate adenosine over a prolonged period of time. EP-A 0970696 teaches the combination of loop diuretics with A₁ adenosine receptor antagonists. US-A 2007/219221 teaches using A_{2B} adenosine receptor antagonists. WO 2001/058241 teaches A_{2A} adenosine receptor antagonists and uses thereof in hepatic applications. WO 2006/115690 refers to pharmaceutical emulsions having adjusted pH values. WO 2003/022284 teaches methods for treating pulmonary disease by means of with A₁ adenosine receptor antagonists.

Recent studies have speculated that non-alcoholic fatty liver disease may represent the end result of several diverse insults. These reports imply that the pathogenesis of non-alcoholic fatty liver disease may be multifactorial and the mechanisms underlying this entity include an amino acid imbalance and endotoxemia associated with overwhelming infection or starvation-associated bacterial translocation. Most recently, studies have suggested that this entity may be due most commonly to hyperinsulinemia and insulin resistance.

The materials, methods and examples are only illustrative and are not intended to be limiting. The citation of references herein shall not be construed as an admission that such is prior art to the present invention.

### SUMMARY OF THE INVENTION

The scope of the invention is defined by the claims. Any references in the description to methods of treatment refer to the compounds, pharmaceutical compositions and medicaments of the present invention for use in a method for treatment of the human (or animal) body by therapy (or for diagnosis).

The invention provides an A₁ or an A_{2B} adenosine receptor antagonist for use in a method of inhibiting the accumulation of fatty deposits in the liver.

Other adenosine receptor antagonists and other hepatic diseases are also discussed in the present disclosure but only form part of the invention in so far as they fall within the scope of the claims.

It is disclosed that the adenosine receptor may be selected from the group consisting of A₁, A_{2A}, A_{2B} and A₃. In a particular embodiment, the adenosine receptor is an A₁ or A_{2B} receptor, and the antagonist is an adenosine A₁ or A_{2B} receptor antagonist. In yet another embodiment, the adenosine receptor antagonist inhibits more than one adenosine receptor.

In a more particular embodiment, the agent that antagonizes an adenosine receptor is an adenosine A₁ receptor antagonist or an adenosine A_{2B} receptor antagonist. The adenosine receptor antagonist may be a small organic molecule, a protein or peptide, a nucleic acid or an antibody.

In yet another more particular embodiment, the A₁ or A_{2B} adenosine receptor antagonist is selected from the group consisting of those antagonists effective to antagonize the A₁ receptor including, for instance, theophylline, caffeine, DPCPX (1,3 dipropyl-8-cyclopentylxantine), N-0861, CVT-124, KW-3902, FR166124, FK453, WRC-0571, CPX, FSCPX and others well known in the art; those antagonists effective to antagonize the A_{2A} receptor including, for instance, SCH58261, SM241385, CSC, KF17837, KW6002, ZM 241385 (-(2-[7-amino-2-(2-furyl)[1,2,4]triazolo[2,3-a][1,3,5]triazin-5-ylamino]ethyl)phenol) and others well known in the art; those antagonists effective to antagonize the A_{2B} receptor including, for instance, enprofylline (3-propylxanthine), IPDX, MRS1754, MRS1706 (N-(4-aceptylphenyl)-2-[4-(2,3,6,7-tetrahydro-2,6-dioxo-1,3-dipropyl-1H-purine-8-yl) phenoxoy]acetamide) and others well known in the art; and those antagonists effective to antagonize the A₃ receptor including, for instance, MRS1191 (3-ethyl-5-benzyl-2-methyl-4-phenylethynyl-6-phenyl-1,4-(±)-diphydropyridine-3,5-dicarboxylate), MRS1523, MRE3008F20, MRS1067, MRS1097, L-249313, L-268605, CGS15943, KF26777 and others well known in the art.

In yet another particular embodiment, the A₁ or A_{2B} adenosine receptor antagonist is administered orally or parenterally. It may be administered intravenously, subcutaneously, intramuscularly, or intranasally, or it may be administered via an implanted device, or may be administered to a mucous membrane. The implanted device may be an osmotic pump. The mucous membrane may be the oral or nasal mucosa selected from the group consisting of the buccal mucosa, the sublingual mucosa, the sinusoidal mucosa, the gum, and the inner lip.

The present application teaches a method for treating hepatic disease or a hepatic condition in a mammal comprising administering to the mammal an effective amount of an A₁ or an A_{2B} adenosine receptor antagonist in combination with a therapeutically effective amount of one or more other compounds effective for treating hepatic disease or condition.

In one particular embodiment, an effective amount of an A₁ or an A_{2B} adenosine receptor antagonist may be used in combination with one or more drugs useful in treating a hepatic disease, useful for reducing, inhibiting or preventing fat deposition in the liver, or useful in reducing serum triglycerides, cholesterol or lipids, or a combination of any of these agents.

In another more particular embodiment, the drug is selected from the group consisting of those effective for treating fatty liver and fibrosis including tyrosine kinase inhibitors such as Gleevec and others known in the art, and those effective for treating fatty liver including thiazolidinedione agents such as pioglitazone, rosiglitazone and troglitazone, for instance.

The A₁ or an A_{2B} adenosine receptor antagonist may be administered alone or in combination with one or more of any of the above-noted compounds or agents for treating hepatic disease or condition. The A₁ or A_{2B} adenosine receptor antagonist may be administered with a second A₁ or A_{2B} adenosine receptor antagonist or with a less selective adenosine receptor antagonist. (ie. one that binds other adenosine receptors in addition to A₁ or A_{2B}, for example A_{2A} or A₃).

A further aspect of the invention provides a pharmaceutical composition comprising an A₁ or an A_{2B} adenosine receptor antagonist. The pharmaceutical composition is useful for inhibiting progression of hepatic disease, or for treating, ameliorating, or preventing hepatic disease or a condition associated with hepatic disease, and a pharmaceutically acceptable carrier.

In one embodiment, the A₁ or A_{2B} adenosine receptor antagonist may be selective for the receptor, or it may be a non-selective adenosine receptor antagonist, which may block or inhibit one or more of the following receptors: A₁, A_{2A}, A_{2B} or A₃. In a preferred embodiment, the adenosine receptor antagonist is an adenosine A₁ or A_{2B} receptor antagonist.

In another particular embodiment, the pharmaceutical composition comprises the A₁ or A_{2B} adenosine receptor antagonist alone or in combination with one or more compounds or agents effective for inhibiting progression of hepatic disease, or for treating, ameliorating, or preventing hepatic disease or a condition associated with hepatic disease. The A₁ or A_{2B} adenosine receptor antagonist and the one or more compounds or agents may be formulated and administered alone or together. The pharmaceutical compositions comprising the A₁ or A_{2B} adenosine receptor antagonist and the one or more compounds or agents may be administered concurrently or sequentially. In another particular embodiment, the one or more compounds or agents effective for treating hepatic disease are selected from the group consisting of those effective for treating fatty liver and fibrosis including tyrosine kinase inhibitors such as Gleevec and others known in the art, and those effective for treating fatty liver including thiazolidinedione agents such as pioglitazone, rosiglitazone and troglitazone, for instance. The pharmaceutical compositions may be delivered orally or parenterally. They may be delivered via the intravenous route, the intramuscular route, or the subcutaneous route. They may be delivered as an immediate release formulation or as a slow or sustained release formulation.

In another more particular embodiment, the pharmaceutical composition comprising the A₁ or A_{2B} adenosine receptor antagonist may also contain one or more drugs selected from the group consisting of those effective for treating fatty liver and fibrosis including tyrosine kinase inhibitors such as Gleevec and others known in the art, and those effective for treating fatty liver including thiazolidinedione agents such as pioglitazone, rosiglitazone and troglitazone, for instance.

Other objects and advantages will become apparent to those skilled in the art from a review of the following description which proceeds with reference to the following illustrative drawings.

### BRIEF DESCRIPTION OF THE DRAWINGS

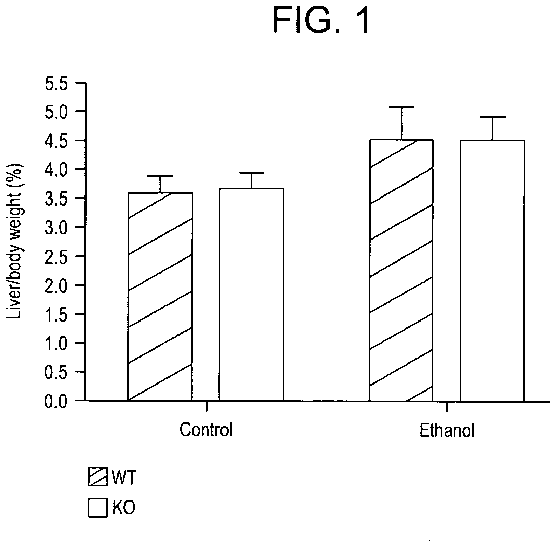

FIG. 1 demonstrates liver weight increase in ethanol-treated mice. After sacrifice of the mice liver weight was measured, and liver/body weight ratios were calculated. The liver/body weight ratio had significantly more increase in ethanol, fatty diet-treated-mice compared with control mice (P<0.01), but no difference was observed between WT (wild type) and KO (knock out) mice either after a normal mouse diet or following six weeks of a high fat, ethanol-containing diet.

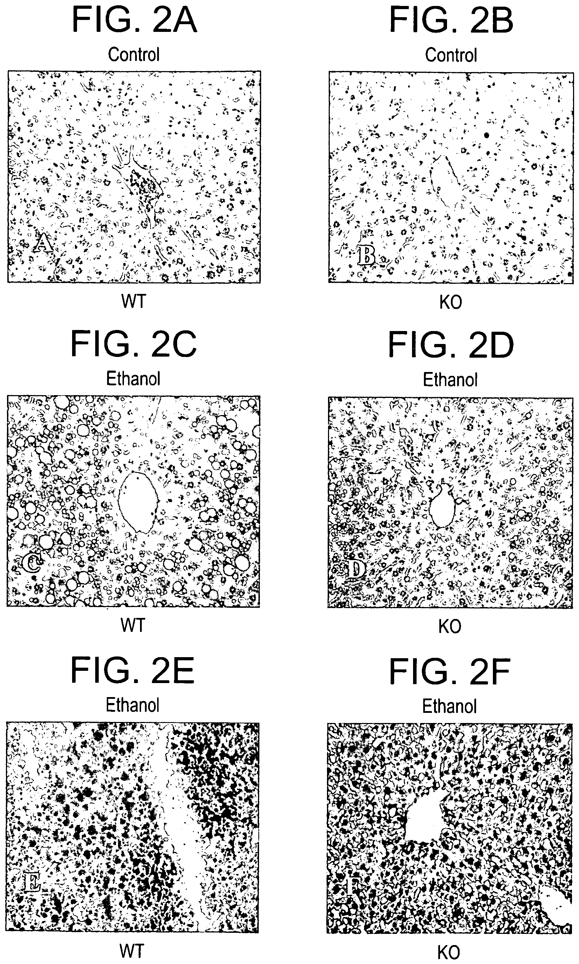

FIG. 2 demonstrates that hepatic steatosis is less marked in A₁ knockout than wild type (WT) mice treated with alcohol. Severe hepatic steatosis was observed in WT mice following ethanol treatment for six weeks, as demonstrated by both H&E staining and Oil Red O staining. Liver sections from (A) WT mouse treated with normal diet (H&E stain); (B) A₁ receptor KO mouse treated with normal diet (H&E Stain); (C) WT mouse treated with high fat/ethanol containing diet (H&E stain); (D) A₁ knockout mouse treated with high fat/ethanol containing diet (H&E Stain); (E) WT mouse treated with high fat/ethanol containing diet (Oil Red O stain); and (F) A₁ knockout mouse treated with high fat/ethanol containing diet (Oil Red O Stain).

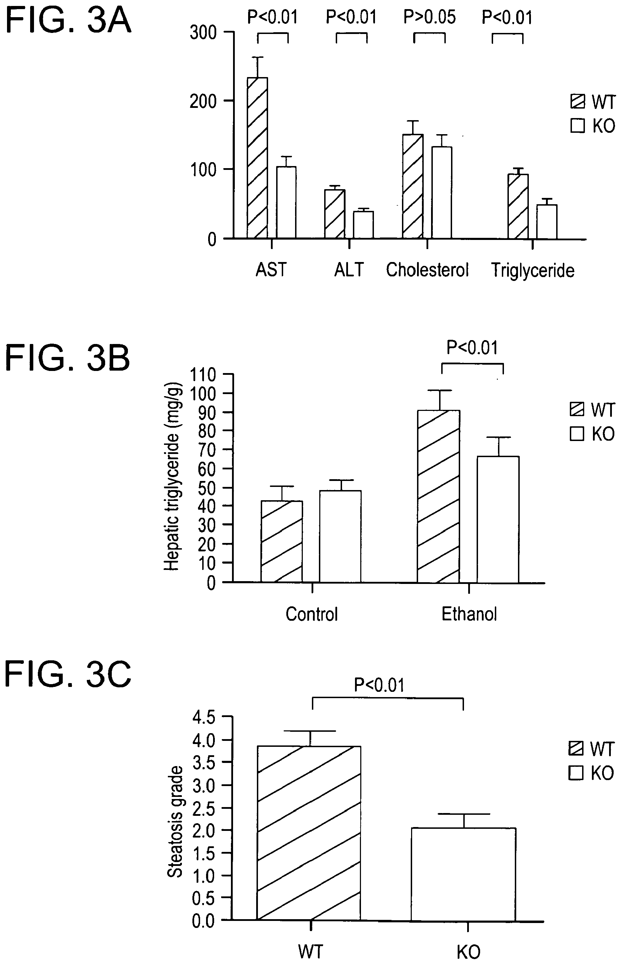

FIG. 3 demonstrates lower serum AST, ALT and triglycerides and diminished hepatic triglyceride content and steatosis scores in high fat/ethanol containing diet-treated A₁ KO than WT mice. (A) Serum AST, ALT and triglyceride were significantly higher in WT mice than these in KO mice following high fat/ethanol treatment for 6 weeks (AST: 232.0 ± 30.9 UI/L vesus 103.3 ± 15.5 UI/L; ALT: 69.5 ± 5.97 UI/L versus 38.3 ± 5.1 UI/L; triglycerides 92.5 ± 9.2 mg/dl versus 49.3 ± 9.4 mg/dl; n=6 in each group, *P*<0.01 respectively). (B) Hepatic triglyceride was increased in high fat/ethanol-treated mice compared to control diet-treated mice, but there was much greater increase in WT than KO mice following treatment with high fat/ethanol diet animals (90.6 ± 11.0 mg/g versus 66.8 ± 10.5 mg/g, n=6 in each group, *P*<0.01). (C) Hepatic steatosis score was significantly higher in WT mice than in KO mice treated with high fat/ethanol diet (3.83 ± 0.39 versus 2.08 ± 0.32, n=6 in each group, *P*<0.01).

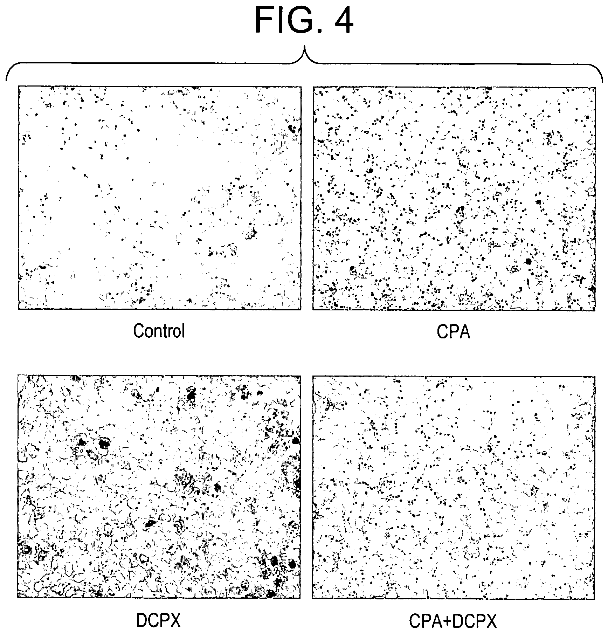

FIG. 4 demonstrates that an adenosine A₁ receptor agonist increased hepatocellular fat content. Adenosine receptor A₁ agonist CPA significantly increased the lipid content of hepatocytes and its effect was nearly completely blocked by the adenosine A₁ receptor antagonist DCPX.

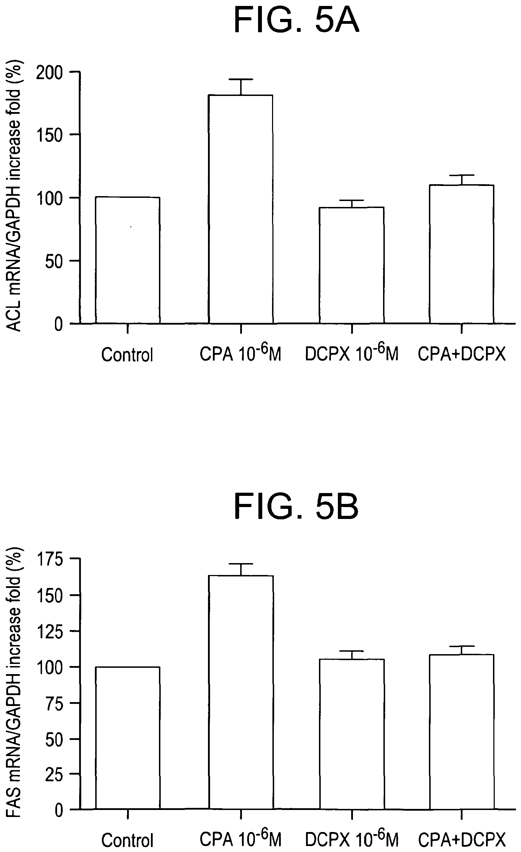

FIG. 5 demonstrates that an adenosine A₁ receptor agonist increased lipid synthesis associated enzymes. mRNA for ACL and FAS were significantly increased following CPA treatment for 24 hours and this increase was completely blocked by DPCPX (ACL: control 100.0%, CPA 181.4 ± 12.9%, DCPX 92.0 ± 5.2%, CPA+DCPX 110.0 ± 7.5%; FAS: control 100.0%, CPA 164.2 ± 6.8%, DCPX 105.8 ± 5.3%, CPA+DCPX 108.3 ± 5.6%, n=3 in each group, CPA versus any other group, P<0.01 respectively).

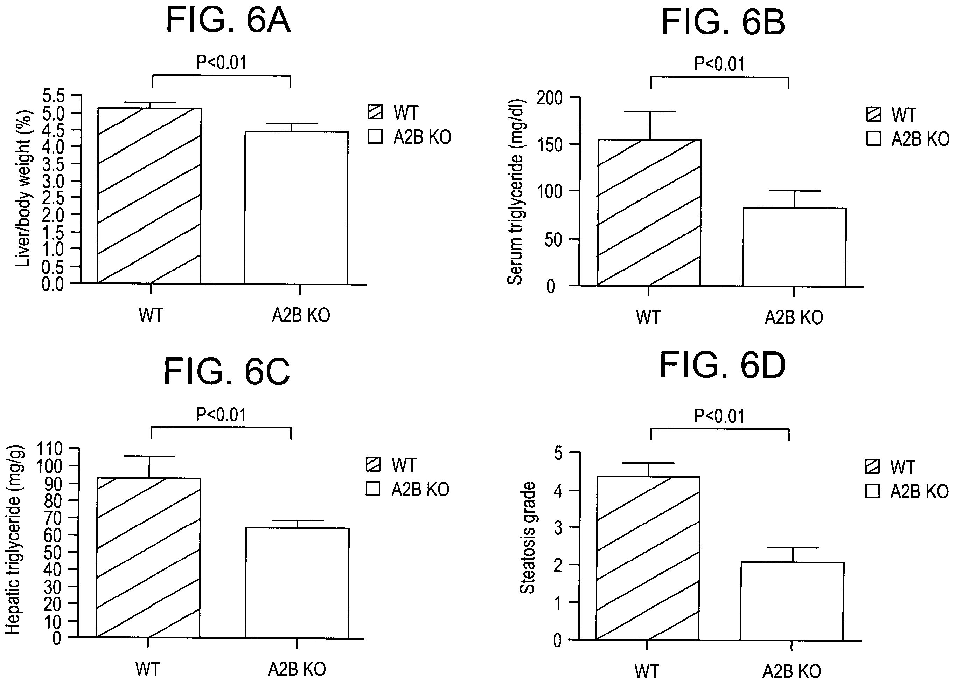

FIG. 6 demonstrates less liver weight, triglyceride and steatosis grade in ethanol-treated A_{2B} knockout mice. (A) In the end of the experiment, liver weight was measured, and liver/body weight ratios were calculated. The liver/body weight ratio (%) was much less in ethanol treated A_{2B} KO mice compared with wild-type mice (4.46 ± 0.25% versus 5.09 ± 0.19%, n=4 in each group, *P*<0.01). (B) Serum triglycerides were significantly higher in WT mice than those in A2BKO mice following ethanol treatment for 6 weeks (154.83 ± 30.56 mg/dl versus 82.0 ± 20.27 mg/dl; n=4 in each group, *P*<0.01). (C) Hepatic triglyceride also was more increased in WT mice than that in A2BKO mice in ethanol-treated animals (92.98 ± 12.34 mg/g versus 64.41 ± 5.10 mg/g, n=4 in each group, *P*<0.01). (D) The hepatic steatosis score was higher in WT mice than that in A2BKO mice in ethanol-treated animals (4.33 ± 0.39 versus 2.10 ± 0.41, n=4 in each group, P<0.01).

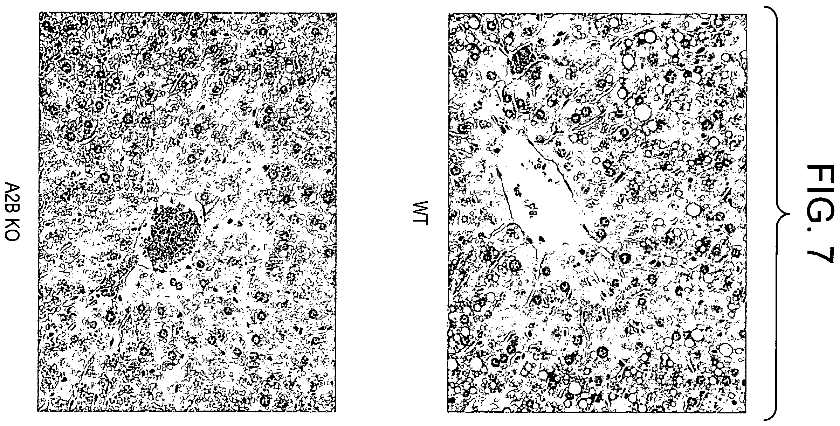

FIG. 7 demonstrates less hepatic steatosis in A_{2B} knockout mice. Severe hepatic steatosis was observed in WT mice following ethanol treatment for 6 weeks in H&E staining.

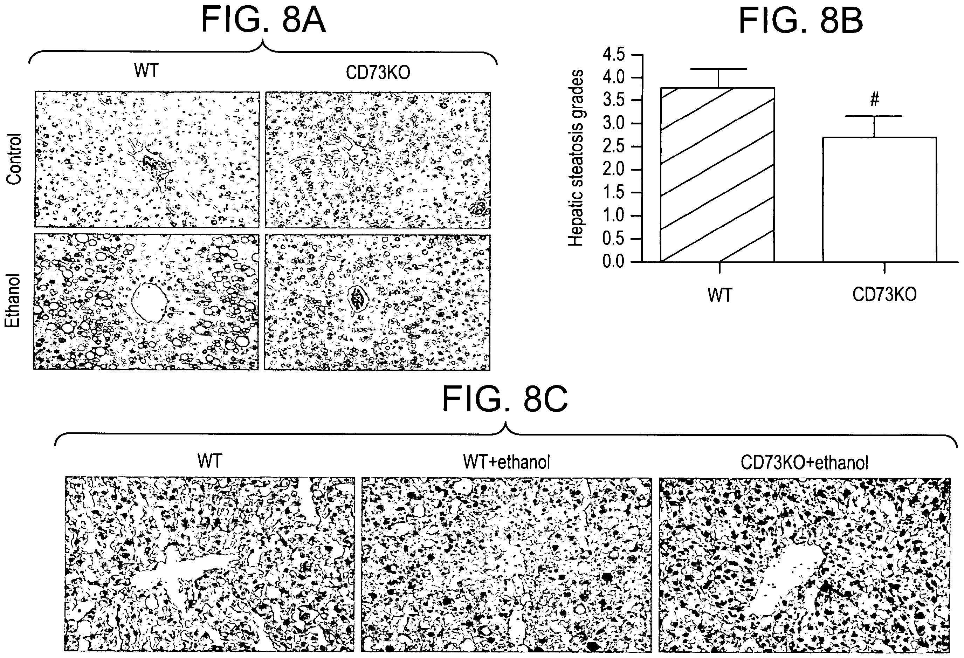

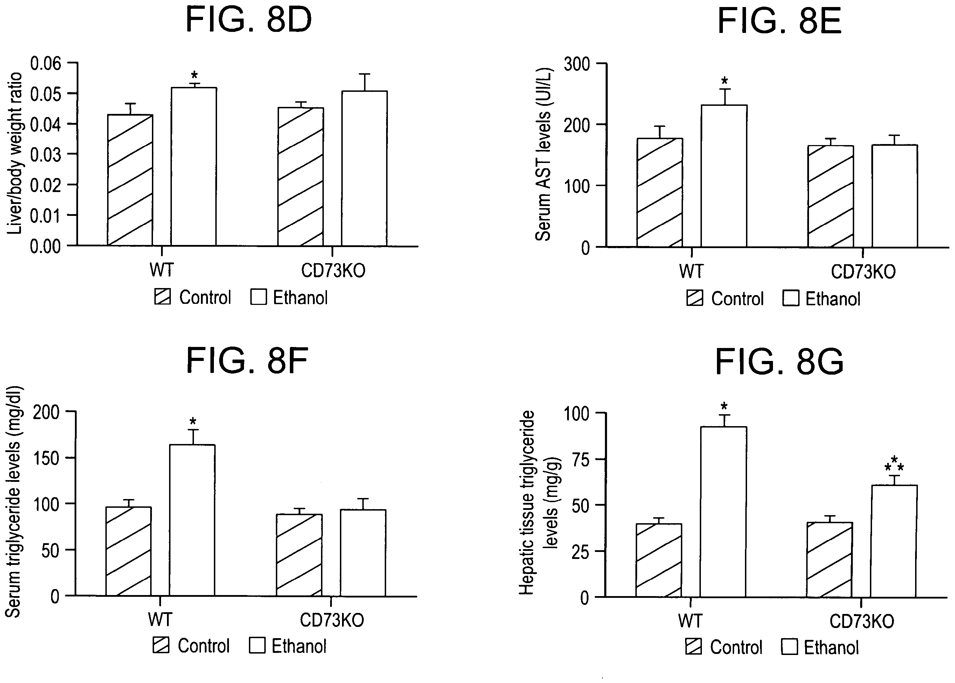

FIG. 8 demonstrates that deletion of ecto-5'Nucleotidase (CD73) prevents the development of ethanol-induced fatty liver in mice. The hepatic steatosis grade was based on the percentage of steatotic hepatocytes in the H&E stained liver sections. Figure 8A provides H&E stained liver sections from maltose and ethanol-treated WT and CD73KO mice (Original magnification ×400). Figure 8B demonstrates hepatic steatosis grades of ethanol-treated WT and CD73KO mice (Original magnification ×400). Steatosis grades in maltose-treated WT and CD73KO mice were 0. Figure 8C demonstrates Oil Red O stained liver sections (Original magnification x400) from ethanol-treated WT and CD73KO mice. Figure 8D demonstrates liver/body ratio of CD73KO and WT mice. Figure 8E demonstrates serum AST levels of WT and CD73KO mice. Figure 8F demonstrates serum triglyceride levels of WT and CD73KO mice. Figure 8G demonstrates hepatic tissue triglyceride levels in WT and CD73KO mice.

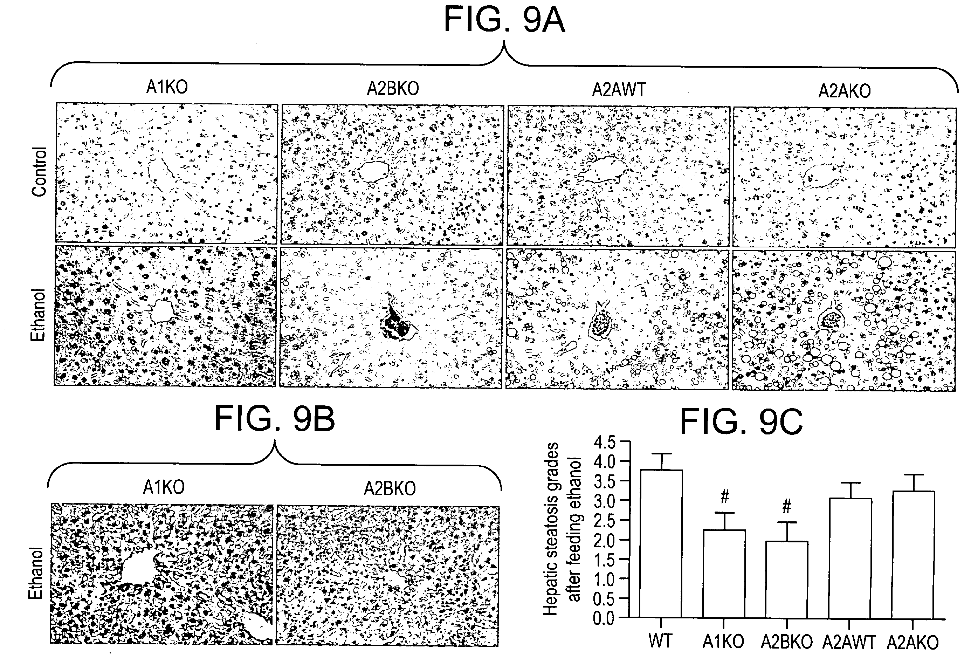

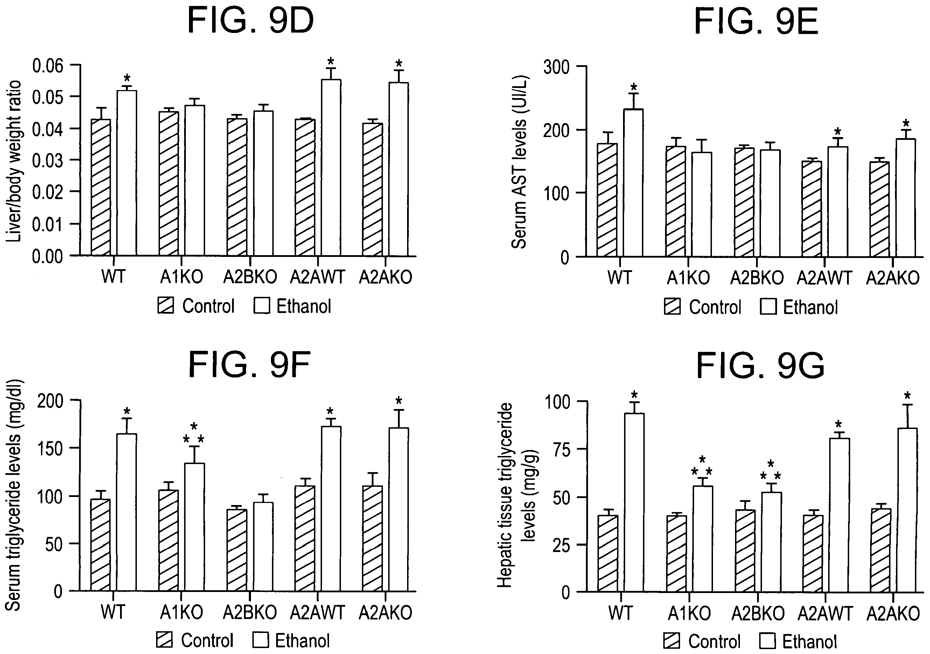

FIG. 9 demonstrates that deletion of adenosine A₁ or A_{2B} receptors protects mice from developing ethanol-induced fatty liver. Figure 9A demonstrates H&E stained liver sections from maltose and ethanol-treated A1KO, A2BKO, A_{2A} KO and WT mice (Original magnification × 400). Figure 9B demonstrates H&E stained liver sections from maltose and ethanol- treated A2AKO and WT mice (Original magnification × 400). Figure 9B demonstrates Oil Red O stained liver sections (Original magnification x 400) from ethanol-treated A1KO and A2BKO mice. Figure 9C demonstrates hepatic steatosis grades of ethanol-treated A1KO, A2BKO, A2AKO and WT mice (Original magnification × 400). Steatosis grades in maltose-treated A1KO, A2BKO and A2AKO mice were 0. Figure 9D demonstrates Liver/body ratio of A1KO, A2BKO, A2AKO and WT mice. Figure 9E demonstrates serum AST levels of A1KO, A2BKO and WT mice. Figure 9F demonstrates serum triglyceride levels of A1KO, A2BKO, A2AKO and WT mice. Figure 9G demonstrates hepatic tissue triglyceride levels in A1KO, A2BKO, A2AKO and WTmice. (#A1KO mice or A2BKO mice versus WT mice, *P*<0.01, respectively; *ethanol mice vs control mice in different groups, respectively, *P*<0.01; **ethanol KO mice versus control WT mice or ethanol WT mice, P<0.01, respectively. N=5-10.)

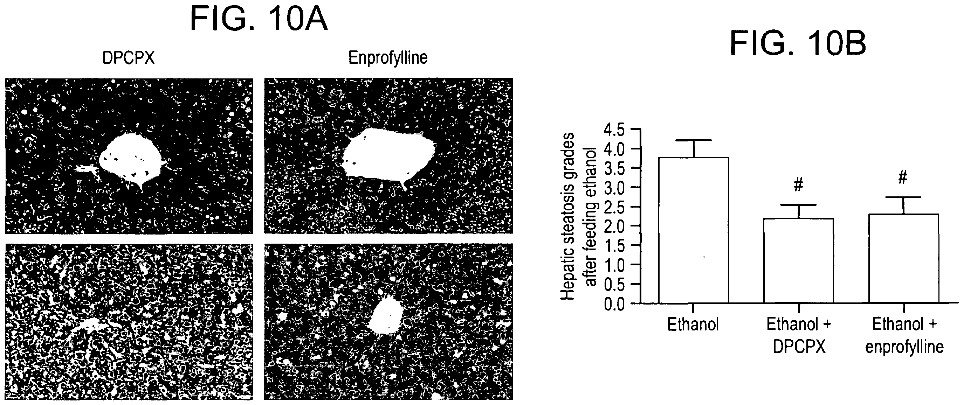

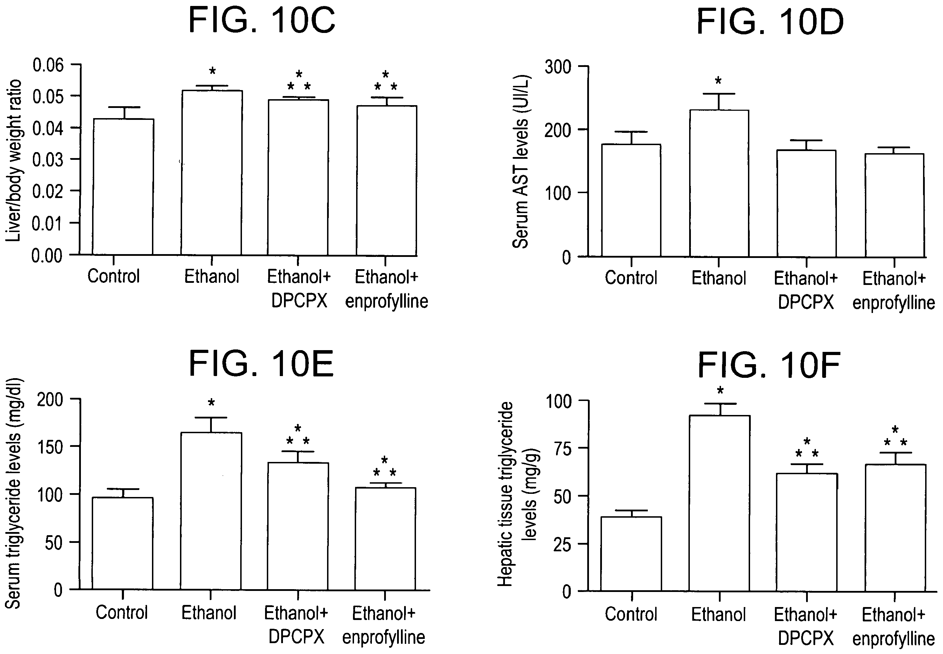

FIG. 10 demonstrates that blockade of adenosine A₁ or A_{2B} receptors protects mice from developing ethanol-induced fatty liver. Figure 10A demonstrates H&E and Oil Red O staining of liver sections of DPCPX- or enprofylline-treated mice (Original magnification × 400). Figure 10B demonstrates hepatic steatosis grades. Figure 10C demonstrates the liver/ body ratio. Figure 10D demonstrates serum AST levels. Figure 10E provides serum triglyceride levels. Figure 10F provides hepatic tissue triglyceride levels. (*Drug-treated ethanol mice versus ethanol mice in different groups, *P*<0.01, respectively; **drug-treated ethanol mice versus control mice in different groups, *P*<0.01 or *P*<0.05, respectively. N=5-10 in each group.)

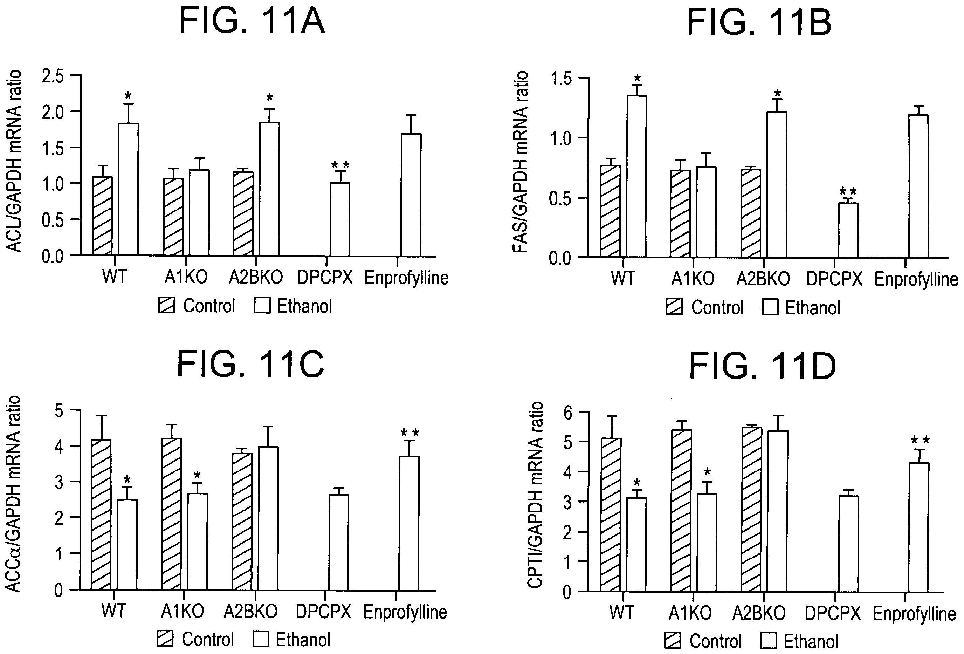

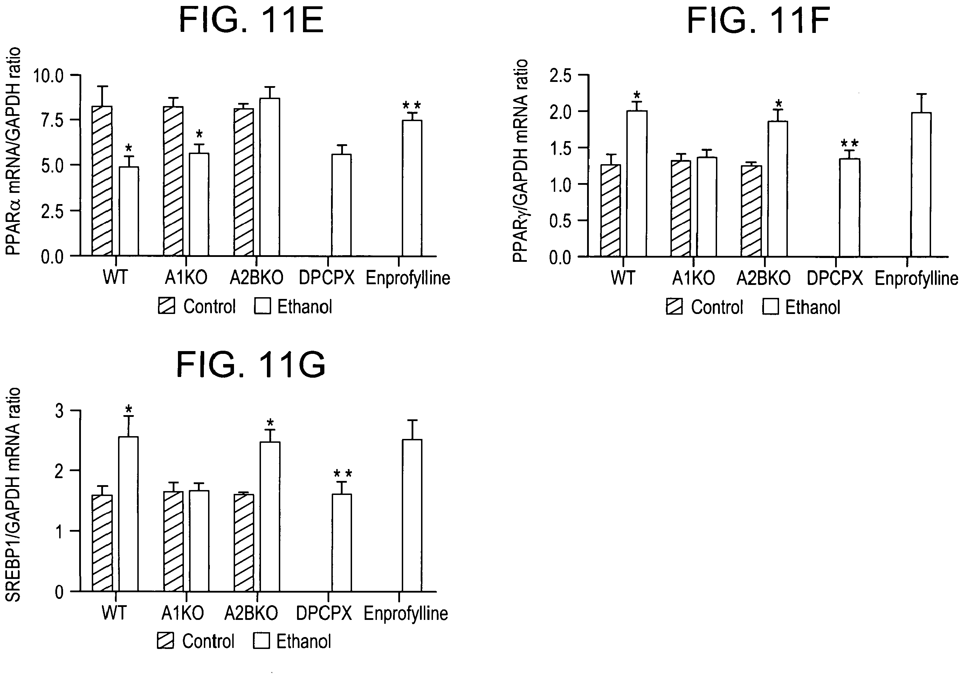

FIG. 11 demonstrates that ethanol ingestion modulates expression of hepatic transcription factors SREBP1 and PPARα and hepatic enzymes and transporters involved in fatty acid synthesis, metabolism and transport. Figure 11A demonstrates mRNA expression of ACL. Figure 11B demonstrates mRNA expression of FAS. Figure 11C demonstrates mRNA expression of ACCα. Figure 11D demonstrates mRNA expression of CPTI. Figure 11E demonstrates mRNA expression of PPARα. Figure 11F demonstrates mRNA expression of PPARγ. Figure 11G demonstrates mRNA expression of SREBP1. (*Ethanol versus control in different groups, P<0.01, respectively; **DPCPX or enprofylline-treated mice versus ethanol WT mice, P<0.01, respectively, n=5 in each group).

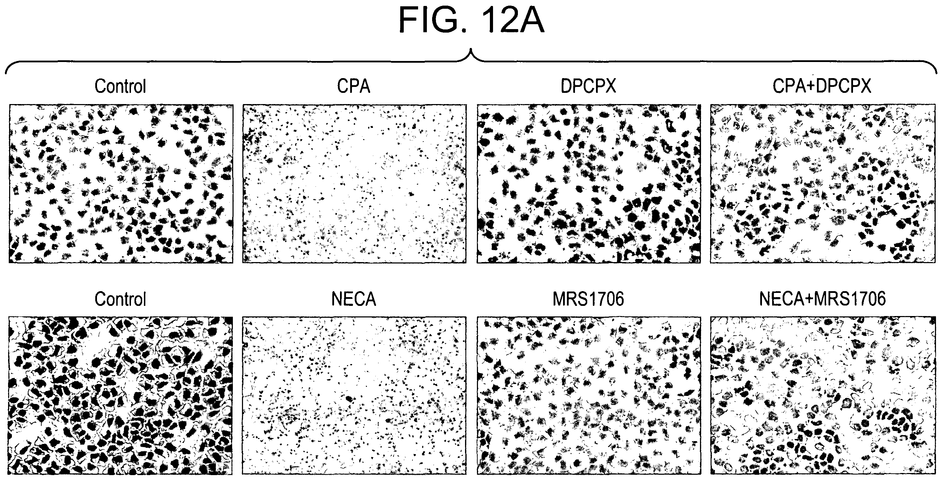

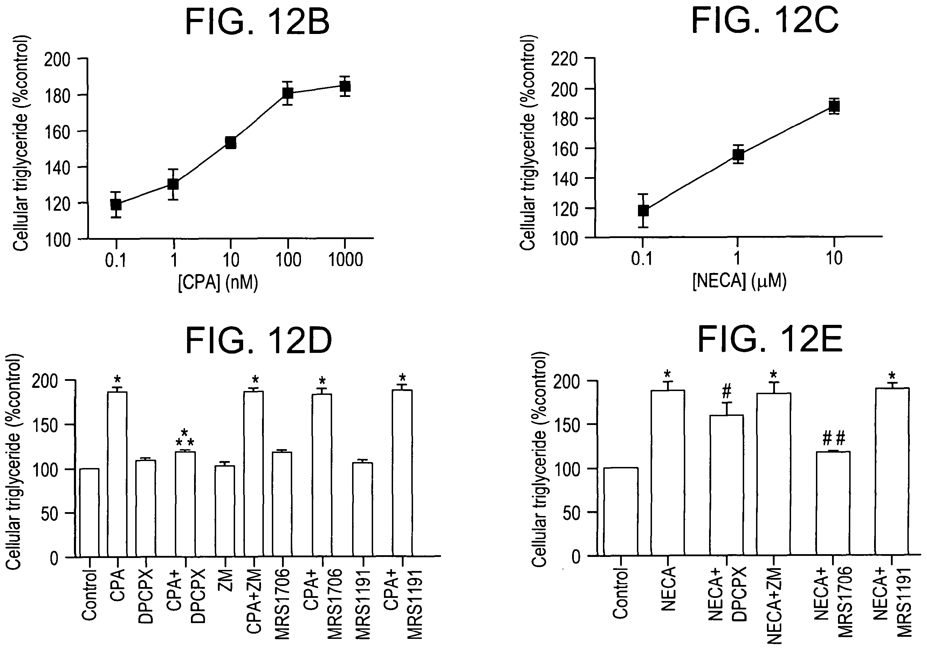

FIG. 12 demonstrates that agonists of adenosine A₁ and A_{2B} receptors promote fat accumulation in a cultured murine hepatocyte cell line. Figure 12A demonstrates Oil red O staining of AML-12 hepatocytic cells (Original magnification ×400). Figure 12 B demonstrates cellular triglyceride content of AML-12 cells. The data are expressed as percentages of control (mean ±SD) from four independent experiments (*Treatment vscontrol, P<0.01; **antagonist or agonist + antagonist vs agonist, P<0.01, respectively).

### DETAILED DESCRIPTION OF THE INVENTION

Before the present methods and treatment methodologies are described, it is to be understood that this invention is not limited to particular methods and experimental conditions described, as such methods and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein and/or which will become apparent to those persons skilled in the art upon reading this disclosure and so forth in their entirety.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

In accordance with the present invention there may be employed conventional molecular biology, microbiology, and recombinant DNA techniques within the skill of the art. Such techniques are explained fully in the literature. *See, e.g.,* Sambrook et al., "Molecular Cloning: A Laboratory Manual" (1989); "Current Protocols in Molecular Biology" Volumes I-III [Ausubel, R. M., ed. (1994)]; "Cell Biology: A Laboratory Handbook" Volumes I-III [J. E. Celis, ed. (1994))]; "Current Protocols in Immunology" Volumes I-III [Coligan, J. E., ed. (1994)]; "Oligonucleotide Synthesis" (M. J. Gait ed. 1984); "Nucleic Acid Hybridization" [B. D. Hames & S. J. Higgins eds. (1985)]; "Transcription And Translation" [B. D. Hames & S. J. Higgins, eds. (1984)]; "Animal Cell Culture" [R. I. Freshney, ed. (1986)]; "Immobilized Cells And Enzymes" [IRL Press, (1986)]; B. Perbal, "A Practical Guide To Molecular Cloning" (1984).

The terms used herein have the meanings recognized and known to those of skill in the art, however, for convenience and completeness, particular terms and their meanings are provided below.

"Agent" refers to all materials that may be used to prepare pharmaceutical and diagnostic compositions, or that may be compounds such as small synthetic or naturally derived organic compounds, nucleic acids, polypeptides, antibodies, fragments, isoforms, variants, or other materials that may be used independently for such purposes, all in accordance with the present invention.

"Antagonist" refers to an agent that down-regulates (e.g., suppresses or inhibits) at least one bioactivity of a protein. An "antagonist" or an agent that "antagonizes" may be a compound which inhibits or decreases the interaction between a protein and another molecule, e.g., a target peptide or enzyme substrate. An antagonist may also be a compound that down-regulates expression of a gene or which reduces the amount of expressed protein present. Methods for assessing the ability of an agent to "antagonize" or "inhibit" an adenosine receptor are known to those skilled in the art.

"Analog" as used herein, refers to a chemical compound, a nucleotide, a protein, or a polypeptide that possesses similar or identical activity or functions as the chemical compounds, nucleotides, proteins or polypeptides having the desired activity and therapeutic effect of the present invention (e.g. to treat hepatic disease), but need not necessarily comprise a compound that is similar or identical to those compounds of the preferred embodiment, or possess a structure that is similar or identical to the agents of the present invention.

"Derivative" refers to the chemical modification of molecules, either synthetic organic molecules or proteins, nucleic acids, or any class of small molecules such as fatty acids, or other small molecules that are prepared either synthetically or isolated from a natural source, such as a plant, that retain at least one function of the active parent molecule, but may be structurally different. Chemical modifications may include, for example, replacement of hydrogen by an alkyl, acyl, or amino group. It may also refer to chemically similar compounds which have been chemically altered to increase bioavailability, absorption, or to decrease toxicity. A derivative polypeptide is one modified by glycosylation, pegylation, or any similar process that retains at least one biological or immunological function of the polypeptide from which it was derived.

A "small molecule" refers to a composition having a molecular weight of less than 3 kilodaltons (kDa), preferably less than 1.5 kilodaltons, more preferably less than about 1 kilodalton. Small molecules may be nucleic acids, peptides, polypeptides, peptidomimetics, carbohydrates, lipids or other organic (carbon-containing) or inorganic molecules. As those skilled in the art will appreciate, based on the present description, extensive libraries of chemical and/or biological mixtures, often fungal, bacterial, or algal extracts, may be screened with any of the assays of the invention to identify compounds that modulate a bioactivity. A "small organic molecule" is an organic compound (or organic compound complexed with an inorganic compound (e.g., metal)) that has a molecular weight of less than 3 kilodaltons, and preferably less than 1.5 kilodaltons, and more preferably less than about 1 kDa.

"Diagnosis" or "screening" refers to diagnosis, prognosis, monitoring, characterizing, selecting patients, including participants in clinical trials, and identifying patients at risk for or having a particular disorder or clinical event or those most likely to respond to a particular therapeutic treatment, or for assessing or monitoring a patient's response to a particular therapeutic treatment.

"Combination therapy" is well known in current medical practice. Treatment by combining two or more agents that target the same pathogen or biochemical pathway sometimes results in greater efficacy and diminished side effects relative to using the therapeutically relevant dose of each agent alone. In some cases, the efficacy of the drug combination is additive (the efficacy of the combination is approximately equal to the sum of the effects of each drug alone), but in other cases the effect can be synergistic (the efficacy of the combination is greater than the sum of the effects of each drug given alone). As used herein, the term "combination therapy" means the two compounds can be delivered in a simultaneous manner, i.e. concurrently, or one of the compounds may be administered first, followed by the second agent, i.e. sequentially. The desired result can be either a subjective relief of one or more symptoms or an objectively identifiable improvement.

"Modulation" or "modulates" or "modulating" refers to up regulation (i.e., activation or stimulation), down regulation (i.e., inhibition or suppression) of a response, or the two in combination or apart. As used herein, an adenosine receptor "modulator" or "modulating" compound or agent is a compound or agent that modulates at least one response involved in the pathogenesis or progression of hepatic disease.

As used herein, the term "candidate compound" or "test compound" or "agent" or "test agent" refers to any compound or molecule that is to be tested. The terms, which are used interchangeably, refer to biological or chemical compounds such as simple or complex organic or inorganic molecules, peptides, proteins, oligonucleotides, polynucleotides, carbohydrates, or lipoproteins. A vast array of compounds can be synthesized, for example oligomers, such as oligopeptides and oligonucleotides, and synthetic organic compounds based on various core structures, and these are also included in the terms noted above. In addition, various natural sources can provide compounds for screening, such as plant or animal extracts, and the like. Compounds can be tested singly or in combination with one another. Agents or candidate compounds can be randomly selected or rationally selected or designed. As used herein, an agent or candidate compound is said to be "randomly selected" when the agent is chosen randomly without considering the specific interaction between the agent and the target compound or site. As used herein, an agent is said to be "rationally selected or designed", when the agent is chosen on a nonrandom basis which takes into account the specific interaction between the agent and the target site and/or the conformation in connection with the agent's action.

"Treatment" or "treating" refers to therapy, prevention and prophylaxis and particularly refers to the administration of medicine or performing medical procedures with respect to a patient, for either prophylaxis (prevention) or to cure or reduce the extent of or likelihood of occurrence of the infirmity or malady or condition or event. In the present invention, the treatments using the agents described may be provided to treat hepatic disease. Most preferably, treatment is for the purpose of reducing or diminishing the symptoms or progression of a disease or disorder of the liver. Treating as used herein also means the administration of the compounds for preventing the development of a hepatic disease. Furthermore, in treating a subject, the compounds of the invention may be administered to a subject already suffering from hepatic disease.

"Subject" or "patient" refers to a mammal, preferably a human, in need of treatment for a condition, disorder or disease.

An "amount sufficient to inhibit progression of a hepatic disease" refers to the amount of the receptor antagonist sufficient to prevent or slow deposition of fatty deposits in the liver or the development or progression of fibrosis of the liver.

In a specific embodiment, the term "about" means within 20%, preferably within 10%, and more preferably within 5% and in some instances within 1% or less.

An "effective amount" or a "therapeutically effective amount" is an amount sufficient to decrease or prevent the symptoms associated with the conditions disclosed herein, including diseases affecting the liver as well as an amount sufficient to slow or prevent further pathological change in the morphology of the liver. For example, an "effective amount" for therapeutic uses is the amount of the composition comprising an active compound herein required to provide a clinically significant increase in healing rates or reduction in symptoms or to reduce morphological change including fat deposition or fibrotic formation within the liver.

As used herein, the term "hepatic disease or condition" refers to diseases, conditions, disorders and syndromes having a symptom or pathology involving the liver. Examples of hepatic disease includes pathology secondary to ingestion of hepatotoxic medicines such as chemotherapy and cancer drugs, antibiotics, analgesics, antiemetics, and other medications. Also, hepatic disease includes pathology secondary to ingestion of alcohol and drug use. Other causes of hepatic disease include, but are not limited to, viral and alcoholic hepatitis, Wilson's disease, hemochromatosis, steatosis, and nonalcoholic steatohepatitis (NASH) or nonalcoholic fatty liver disease (NAFD). Individuals who have a hepatic disease can be identified by those having ordinary skill in the art by well known diagnostic means and criteria. Individuals who are susceptible to a disease can be identified by those having ordinary skill in the art based upon family medical history and/or the presence of genetic markers or genes associated with a hepatic disease or the presence of altered serum levels of lipids, cholesterol, iron or triglycerides characteristic of a hepatic disease.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

Binding compounds can also be characterized by their effect on the activity of the target molecule. Thus, a "low activity" compound has an inhibitory concentration (IC₅₀) (for inhibitors or antagonists) or effective concentration (EC₅₀) (applicable to agonists) of greater than 1 µM under standard conditions. By "very low activity" is meant an IC₅₀ or EC₅₀ of above 100 µM under standard conditions. By "extremely low activity" is meant an IC₅₀ or EC₅₀ of above 1 mM under standard conditions. By "moderate activity" is meant an IC₅₀ or EC₅₀ of 200 nM to 1 µM under standard conditions. By "moderately high activity" is meant an IC₅₀ or EC₅₀ of 1 nM to 200 nM. By "high activity" is meant an IC₅₀ or EC₅₀ of below 1 nM under standard conditions. The IC₅₀ (or EC₅₀) is defined as the concentration of compound at which 50% of the activity of the target molecule (e.g., enzyme or other protein) activity being measured is lost (or gained) relative to activity when no compound is present. Activity can be measured using methods known to those of ordinary skill in the art, e.g., by measuring any detectable product or signal produced by occurrence of an enzymatic reaction, or other activity by a protein being measured.

An individual "at risk" may or may not have detectable disease, and may or may not have displayed detectable disease prior to the treatment methods described herein. "At risk" denotes that an individual who is determined to be more likely to develop a symptom based on conventional risk assessment methods or has one or more risk factors that correlate with development of a hepatic disease or condition characterized by fat deposition in the liver or fibrosis of the liver. An individual having one or more of these risk factors has a higher probability of developing a hepatic disease than an individual without these risk factors.

"Prophylactic" or "therapeutic" treatment refers to administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

### General Description

The invention relates to the unexpected finding that blocking the adenosine receptor with agents that are antagonists of the adenosine receptor, particularly A₁ or an A_{2B} adenosine receptor antagonists, leads to or results in inhibition of hepatic disease, fat deposition in the liver and fibrosis of the liver.

Adenosine, a potent endogenous physiological mediator, regulates a wide variety of physiological processes via interaction with one or more of four G protein-coupled receptors (A₁, A_{2A}, A_{2B}, and A₃), expressed on many cell types, including neutrophils, macrophages, fibroblasts, and endothelial cells.

### Selecting Compounds or Agents

Various adenosine receptor antagonists, including those specific for the A₁ receptor, have been identified and are known in the art. Examples of A₁ receptor antagonists in the art include but are not limited to: xanthine and non-xanthine antagonists described in U.S. Pat. Nos. 6,495,687; 6,489,332; 6,117,998; 6,605,601; 5,840,729; 5,786,360; 2005/0245546; 2005/0119258; 2005/0059683; 2005/0187226; 2004/0110774; 2003/0220358; 2003/0045536; 2002/0111333; and 2002/0082269. Particularly preferred are compounds or agents that are selective for the A₁ adenosine receptor.

Likewise, various adenosine receptor antagonists, including those specific for the A_{2B} receptor, have been identified and are known in the art. Examples of A_{2B} receptor antagonists in the art include but are not limited to those described in U.S. Patent Publication Nos. 2007/0219221; 2006/0293283; 2006/0281921; 2006/0159627; 2006/0142309; 2006/0058322; 2005/0261316; 2005/0119271; 2005/0101778; 2005/0038045; 2004/0209899; 2004/0176399; 2004/0053982; 2003/0229106; 2003/0207879; 2003/0162764; 2003/0139428; 2003/0064999; 2002/0072505; and 2002/0002142. Particularly preferred are compounds or agents which are selective for the A_{2B} adenosine receptor.

In addition, various adenosine receptor antagonists, including those specific for the A_{2A} receptor, have been identified and are known in the art. Examples of A_{2A} receptor antagonists in the art include but are not limited to those described in U.S. Patent Publication Nos. 2007/0208040; 2007/0173505; and 2003/0149060.

In addition, various adenosine receptor antagonists, including those specific for the A₃ receptor, have been identified and are known in the art. Examples of A₃ receptor antagonists in the art include but are not limited to those described in U.S. Patent Publication Nos. 2003/0073708 and 2002/0072505.

Based on this discovery, the present application further provides a method of discovering agents or compounds that are effective to treat hepatic disease. Thus, in one embodiment, methods are provided for screening agents or compounds which inhibit the adenosine A₁ or A_{2B} receptor, thereby treating or inhibiting the progression of hepatic disease.

In one embodiment, agents that interact with (e.g., bind to) and block or antagonize an adenosine receptor, in particular, A₁ or A_{2B} (e.g. a functionally active fragment), are identified in a cell-based assay system. In accordance with this embodiment, cells expressing an adenosine receptor, a fragment of an adenosine receptor, an adenosine receptor related polypeptide, or a binding fragment thereof, are contacted with a candidate compound or a control compound and the ability of the candidate compound to interact with the receptor or fragment thereof is determined. Alternatively, the ability of a candidate compound to compete for binding with a known ligand or compound known to bind the receptor is measured. If desired, this assay may be used to screen a plurality (e.g. a library) of candidate compounds. The cell, for example, can be of prokaryotic origin (e.g., *E. coli*) or eukaryotic origin (e.g., yeast, insect or mammalian). Further, the cells can express the receptor endogenously or be genetically engineered to express the receptor, a binding fragment or a receptor fusion protein. In some embodiments, the receptor or fragment thereof, or the candidate compound is labeled, for example with a radioactive label (such as ³²P, ³⁵S or ¹²⁵I) or a fluorescent label (such as fluorescein isothiocyanate, rhodamine, phycoerythrin, phycocyanin, allophycocyanin, o-phthaldehyde or fluorescamine) to enable detection of an interaction between the A₁ or A_{2B} receptor and a candidate compound. The ability of the candidate compound to interact directly or indirectly with a receptor or binding fragment thereof or a fusion protein or to modulate the activity of the receptor can be determined by methods known to those of skill in the art. For example, the interaction or modulation by a candidate compound can be determined by flow cytometry, a scintillation assay, immunoprecipitation or western blot analysis, based on the present description, or by a competitive radioreceptor assay.

Briefly, selecting the compounds that interact with or bind to an adenosine receptor or otherwise inhibit or block the receptor may be performed in multiple ways. The compounds may first be chosen based on their structural and functional characteristics, using one of a number of approaches known in the art. For instance, homology modeling can be used to screen small molecule libraries in order to determine which molecules would be candidates to interact with the receptor thereby selecting plausible targets. The compounds to be screened can include both natural and synthetic ligands. Furthermore, any desired compound may be examined for its ability to interact with or bind to the receptor including as described below.

Binding to or interaction with adenosine receptors may be determined by performing an assay such as, e.g., a binding assay between a desired compound and an adenosine receptor. In one embodiment, this is done by contacting said compound to an adenosine receptor and determining its dissociation rate. Numerous possibilities for performing binding assays are well known in the art. The indication of a compound's ability to bind to an adenosine receptor is determined, e.g., by a dissociation rate, and the correlation of binding activity and dissociation rates is well established in the art. For example, the assay may be performed by radio-labeling a reference compound, or other suitable radioactive marker, and incubating it with the cell bearing an adenosine receptor, in particular, A₁ or A_{2B}. Test compounds are then added to these reactions in increasing concentrations. After optimal incubation, the reference compound and receptor complexes are separated, e.g., with chromatography columns, and evaluated for bound ¹²⁵I-labeled peptide with a gamma (γ) counter. The amount of the test compound necessary to inhibit 50% of the reference compound's binding is determined. These values are then normalized to the concentration of unlabeled reference compound's binding (relative inhibitory concentration (RIC)⁻¹=concentrationₜₑₛₜ/concentration_{reference}). A small RIC⁻¹ value indicates strong relative binding, whereas a large RIC.sup.-1 value indicates weak relative binding. See, for example, Latek et al., Proc. Natl. Acad. Sci. USA, Vol. 97, No. 21, pp. 11460-11465, 2000. An adenosine receptor antagonist mimic may be computationally evaluated and designed by means of a series of steps in which chemical groups or fragments are screened and selected for their ability to associate with the individual binding pockets or interface surfaces of the protein (e.g. the A₁ or A_{2B} receptor). One skilled in the art may employ one of several methods to screen chemical groups or fragments for their ability to associate with the adenosine receptor. This process may begin by visual inspection of, for example, the protein/protein interfaces or the binding site on a computer screen based on the available crystal complex coordinates of the receptor, including a protein known to interact with selected fragments or chemical groups may then be positioned in a variety of orientations, or docked, at an individual surface of the receptor that participates in a protein/protein interface or in the binding pocket. Docking may be accomplished using software such as QUANTA and SYBYL, followed by energy minimization and molecular dynamics with standard molecular mechanics forcefields, such as CHARMM and AMBER (AMBER, version 4.0 (Kollman, University of California at San Francisco ©1994); QUANTA/CHARMM (Molecular Simulations, Inc., Burlington, Mass., ©1994)). Specialized computer programs may also assist in the process of selecting fragments or chemical groups. These include: GRID (Goodford, 1985, J. Med. Chem. 28:849-857), available from Oxford University, Oxford, UK; MCSS (Miranker & Karplus, 1991, Proteins: Structure, Function and Genetics 11:29-34), available from Molecular Simulations, Burlington, Mass.; AUTODOCK (Goodsell & Olsen, 1990, Proteins: Structure, Function, and Genetics 8:195-202), available from Scripps Research Institute, La Jolla, Calif.; and DOCK (Kuntz et al., 1982, J. Mol. Biol. 161:269-288), available from University of California, San Francisco, Calif. Once suitable chemical groups or fragments that bind to A₁ have been selected, they can be assembled into a single compound or inhibitor. Assembly may proceed by visual inspection of the relationship of the fragments to each other in the three-dimensional image displayed on a computer screen in relation to the structure coordinates thereof. This would be followed by manual model building using software such as QUANTA or SYBYL. Useful programs to aid one of skill in the art in connecting the individual chemical groups or fragments include: CAVEAT (Bartlett et al., 1989, "CAVEAT: A Program to Facilitate the Structure-Derived Design of Biologically Active Molecules'. In Molecular Recognition in Chemical and Biological Problems', Special Pub., Royal Chem. Soc. 78:182-196), available from the University of California, Berkeley, Calif.; 3D Database systems such as MACCS-3D (MDL Information Systems, San Leandro, Calif.). This area is reviewed in Martin, 1992, J. Med. Chem. 35:2145-2154); and HOOK (available from Molecular Simulations, Burlington, Mass.). Instead of proceeding to build an adenosine receptor antagonist mimic, in a step-wise fashion one fragment or chemical group at a time, as described above, such compounds may be designed as a whole or 'de novo' using either an empty binding site or the surface of a protein that participates in protein/protein interactions or optionally including some portion(s) of a known activator(s). These methods include: LUDI (Bohm, 1992, J. Comp. Aid. Molec. Design 6:61-78), available from Molecular Simulations, Inc., San Diego, Calif.; LEGEND (Nishibata & Itai, 1991, Tetrahedron 47:8985), available from Molecular Simulations, Burlington, Mass.; and LeapFrog (available from Tripos, Inc., St. Louis, Mo.). Other molecular modeling techniques may also be employed in accordance with this invention. See, e.g., Cohen et al., 1990, J. Med. Chem. 33:883-894. See also, Navia & Murcko, 1992, Current Opinions in Structural Biology 2:202-210.

Once a compound has been designed by the above methods, the efficiency with which that compound may bind to or interact with the adenosine receptor protein may be tested and optimized by computational evaluation. Inhibitors may interact with the receptor in more than one conformation that is similar in overall binding energy. In those cases, the deformation energy of binding is taken to be the difference between the energy of the free compound and the average energy of the conformations observed when the inhibitor binds to the receptor protein.

A compound selected for binding to the adenosine receptor may be further computationally optimized so that in its bound state it would preferably lack repulsive electrostatic interaction with the target protein. Such non-complementary electrostatic interactions include repulsive charge-charge, dipole-dipole and charge-dipole interactions. Specifically, the sum of all electrostatic interactions between the inhibitor and the receptor protein when the mimic is bound to it preferably make a neutral or favorable contribution to the enthalpy of binding. Specific computer software is available in the art to evaluate compound deformation energy and electrostatic interaction. Examples of programs designed for such uses include: Gaussian 92, revision C (Frisch, Gaussian, Inc., Pittsburgh, Pa. ©1992); AMBER, version 4.0 (Kollman, University of California at San Francisco ©1994); QUANTA/CHARMM (Molecular Simulations, Inc., Burlington, Mass., ©1994); and Insight II/Discover (Biosym Technologies Inc., San Diego, Calif., ©1994). These programs may be implemented, for instance, using a computer workstation, as are well-known in the art. Other hardware systems and software packages will be known to those skilled in the art.

Once an adenosine receptor modulating compound (preferably an inhibitor) has been optimally designed, for example as described above, substitutions may then be made in some of its atoms or chemical groups in order to improve or modify its binding properties, or its pharmaceutical properties such as stability or toxicity. Generally, initial substitutions are conservative, i.e., the replacement group will have approximately the same size, shape, hydrophobicity and charge as the original group. One of skill in the art will understand that substitutions known in the art to alter conformation should be avoided. Such altered chemical compounds may then be analyzed for efficiency of binding to the receptor by the same computer methods described in detail above.

### Candidate Compounds and Agents

Examples of agents, candidate compounds or test compounds include, but are not limited to, nucleic acids (e.g., DNA and RNA), carbohydrates, lipids, proteins, peptides, peptidomimetics, small molecules and other drugs. In one preferred embodiment, agents can be obtained using any of the numerous suitable approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, 1997, Anticancer Drug Des. 12:145; U.S. Patent No. 5,738,996; and U.S. Patent No. 5,807,683).

Phage display libraries may be used to screen potential ligands or adenosine receptor modulators. Their usefulness lies in the ability to screen, for example, a library displaying a billion different compounds with only a modest investment of time, money, and resources. For use of phage display libraries in a screening process, see, for instance, Kay et al., Methods, 240-246, 2001. An exemplary scheme for using phage display libraries to identify compounds that bind or interact with an adenosine receptor may be described as follows: initially, an aliquot of the library is introduced into microtiter plate wells that have previously been coated with target protein, e.g. A₁ or A_{2B} receptor. After incubation (e.g. 2 hours), the nonbinding phage are washed away, and the bound phage are recovered by denaturing or destroying the target with exposure to harsh conditions such as, for instance pH 2, but leaving the phage intact. After transferring the phage to another tube, the conditions are neutralized, followed by infection of bacteria with the phage and production of more phage particles. The amplified phage are then rescreened to complete one cycle of affinity selection. After three or more rounds of screening, the phage are plated out such that there are individual plaques that can be further analyzed. For example, the conformation of binding activity of affinity-purified phage for the adenosine A₁ receptor may be obtained by performing ELISAs. One skilled in the art can easily perform these experiments. In one embodiment, an A₁ or A_{2B} receptor molecule used for any of the assays may be selected from a recombinant A₁ or A_{2B} receptor protein, or an A₁ or A_{2B} fusion protein, an analog, derivative, or mimic thereof.

Examples of methods for the synthesis of molecular libraries can be found in the art, for example in: DeWitt et al., 1993, Proc. Natl. Acad. Sci. USA 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994, J. Med. Chem. 37:1233.

Libraries of compounds may be presented, e.g., presented in solution (e.g., Houghten, 1992, Bio/Techniques 13:412-421), or on beads (Lam, 1991, Nature 354:82-84), chips (Fodor, 1993, Nature 364:555-556), bacteria (U.S. Patent No. 5,223,409), spores (U.S. Patent Nos. 5,571,698; 5,403,484; and 5,223,409), plasmids (Cull et al., 1992, Proc. Natl. Acad. Sci. USA 89:1865-1869) or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J. Mol. Biol. 222:301-310).

The methods of screening compounds may also include the specific identification or characterization of such compounds, whose hepatocyte modulating potential was determined by the methods described above. If the identity of the compound is known from the start of the experiment, no additional assays are needed to determine its identity. However, if the screening for compounds that modulate the adenosine A₁ receptor is done with a library of compounds, it may be necessary to perform additional tests to positively identify a compound that satisfies all required conditions of the screening process. There are multiple ways to determine the identity of the compound. One process involves mass spectrometry, for which various methods are available and known to the skilled artisan (see for instance neogenesis.com). Neogenesis' ALIS (automated ligand identification system) spectral search engine and data analysis software allow for a highly specific identification of a ligand structure based on the exact mass of the ligand. One skilled in the art can also readily perform mass spectrometry experiments to determine the identity of the compound.

Antibodies, including polyclonal and monoclonal antibodies, particularly anti- A₁ or anti- A_{2B} receptor antibodies and neutralizing antibodies may be useful as compounds to modulate osteoclast differentiation and/or function. These antibodies are available from Upstate Biologicals, Santa Cruz, or they made be prepared using standard procedures for preparation of polyclonal or monoclonal antibodies known to those skilled in the art. Also, antibodies including both polyclonal and monoclonal antibodies, and drugs that modulate the activity of the adenosine receptor and/or its subunits may possess certain diagnostic applications and may for example, be utilized for the purpose of detecting and/or measuring conditions such as bone diseases, bone loss, or osteoclast differentiation and/or function. The adenosine receptor or its subunits may be used to produce both polyclonal and monoclonal antibodies to themselves in a variety of cellular media, by known techniques such as the hybridoma technique utilizing, for example, fused mouse spleen lymphocytes and myeloma cells. Likewise, small molecules that mimic or antagonize the activity(ies) of the A₁ receptor may be discovered or synthesized, and may be used in diagnostic and/or therapeutic protocols.

### Therapeutic and Prophylactic Compositions and Their Use

Candidates for therapy with the agents identified by the methods described herein are patients either suffering from a hepatic disease.

The invention provides means of treatment comprising administering to a subject an effective amount of an agent of the invention. In a preferred embodiment, the compound is substantially purified (e.g., substantially free from substances that limit its effect or produce undesired side-effects). The subject is preferably an animal, including but not limited to animals such as monkeys, cows, pigs, horses, chickens, cats, dogs, etc., and is preferably a mammal, and most preferably human. In one specific embodiment, a non-human mammal is the subject. In another specific embodiment, a human mammal is the subject. Accordingly, the agents identified by the methods described herein may be formulated as pharmaceutical compositions to be used for prophylaxis or therapeutic use to treat these patients.

Various delivery systems are known and can be used to administer a compound of the invention, e.g., encapsulation in liposomes, microparticles, or microcapsules. Methods of introduction can be enteral or parenteral and include but are not limited to intradernal, intramuscular, intraperitoneal, intravenous, subcutaneous, intranasal, epidural, topical and oral routes. The compounds may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent. In a specific embodiment, it may be desirable to administer the pharmaceutical compositions of the invention locally to the area in need of treatment.

Such compositions comprise a therapeutically effective amount of an agent, and a pharmaceutically acceptable carrier. In a particular embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the compound, preferably in purified form, together with a suitable amount of carrier so as to provide the form for proper administration to the subject. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In another embodiment, the compound can be delivered in a vesicle, in particular a liposome (*See, e.g.* Langer (1990) Science 249:1527-1533; Treat et al., in Liposomes in the Therapy of Infectious Disease and Cancer, Lopez-Berestein and Fidler (eds.), Liss, New York, pp. 353-365 (1989); pp. 317-327)

In yet another embodiment, the compound can be delivered in a controlled or sustained release system. In one embodiment, a pump may be used (see Langer, *supra;* Sefton (1987) CRC Crit. Ref. Biomed. Eng. 14:201; Buchwald et al. (1980) Surgery 88:507; Saudek et al. (1989) N. Engl. J. Med. 321:574). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, J. (1983) Macromol. Sci. Rev. Macromol. Chem. 23:61; Levy et al. (1985) Science 228:190; During et al. (1989) Ann. Neurol. 25:351; Howard et al. (1989) J. Neurosurg. 71:105). In yet another embodiment, a controlled release system can be placed in proximity of the therapeutic target, i.e., the airways, thus requiring only a fraction of the systemic dose. Other suitable controlled release systems are discussed in the review by Langer (1990) Science 249:1527-1533.

In a preferred embodiment of the invention, means for use in a method of treating hepatic disease are provided by administering compositions comprising compounds that block or antagonize an A₁ or an A_{2B} adenosine receptor.

The present invention further contemplates therapeutic compositions useful in practicing the therapeutic methods taught herein. A subject therapeutic composition includes, in admixture, a pharmaceutically acceptable excipient (carrier) and one or more of an adenosine A₁ receptor modulators, as described herein as an active ingredient. In a preferred embodiment, the composition comprises one or more compounds or agents capable of blocking or inhibiting the adenosine receptor.

Effects of the compounds or agents of the invention can first be tested for their ability to block or inhibit the adenosine receptor using standard techniques known in the art. More particularly, the selectivity of the compounds for the receptor can be assessed using radioligand binding assays whereby a test or candidate compound can be assayed for its ability to compete for binding to a cell having or expressing the A₁ or A_{2B} adenosine_receptor. Cells can be transfected with the nucleic acid encoding the various adenosine receptors and competitive binding assays with radiolabeled ligands run to evaluate the specificity of the particular candidate compounds. The cDNAs for human A₁ (see GenBank accession number BC026340), A_{2A} (see GenBank accession number NM₀₀₀₆₇₅), A_{2B} (see GenBank accession number NM₀₀₀₆₇₆) or A₃ (see GenBank accession number AY136749 or L22607 or NM₀₀₀₆₇₇) can be used to prepare the nucleic acid constructs for use in these methods.

The present compounds or agents that modulate the adenosine receptor, in particular, the antagonists or inhibitors of the A₁ or A_{2B} receptor, themselves can be used as the sole active agents, or can be used in combination with one or more other active ingredients. In particular, combination therapy using the adenosine receptor antagonists with one or more other agents that have an effect in treating hepatic diseases or conditions characterized by deposition of fat in the liver or fibrosis are contemplated. These agents are known in the art, and can be selected from the group consisting of those effective for treating fatty liver and fibrosis including tyrosine kinase inhibitors such as Gleevec and others known in the art, and those effective for treating fatty liver including thiazolidinedione agents such as pioglitazone, rosiglitazone and troglitazone, for instance.

When contemplating combination therapy with an A₁ or an A_{2B} adenosine receptor antagonist and one or more of the above-noted agents, it is important to assess clinical safety by methods known to those skilled in the art. Appropriate dose titration may be necessary when certain groups of compounds are contemplated for use together.

The compounds or compositions of the invention may be combined for administration with or embedded in polymeric carrier(s), biodegradable or biomimetic matrices or in a scaffold. The carrier, matrix or scaffold may be of any material that will allow composition to be incorporated and expressed and will be compatible with the addition of cells or in the presence of cells. Preferably, the carrier matrix or scaffold is predominantly non-immunogenic and is biodegradable. Examples of biodegradable materials include, but are not limited to, polyglycolic acid (PGA), polylactic acid (PLA), hyaluronic acid, catgut suture material, gelatin, cellulose, nitrocellulose, collagen, albumin, fibrin, alginate, cotton, or other naturally-occurring biodegradable materials. It may be preferable to sterilize the matrix or scaffold material prior to administration or implantation, e.g., by treatment with ethylene oxide or by gamma irradiation or irradiation with an electron beam. In addition, a number of other materials may be used to form the scaffold or framework structure, including but not limited to: nylon (polyamides), dacron (polyesters), polystyrene, polypropylene, polyacrylates, polyvinyl compounds (e.g., polyvinylchloride), polycarbonate (PVC), polytetrafluorethylene (PTFE, teflon), thermanox (TPX), polymers of hydroxy acids such as polylactic acid (PLA), polyglycolic acid (PGA), and polylactic acid-glycolic acid (PLGA), polyorthoesters, polyanhydrides, polyphosphazenes, and a variety of polyhydroxyalkanoates, and combinations thereof. Matrices suitable include a polymeric mesh or sponge and a polymeric hydrogel. In the preferred embodiment, the matrix is biodegradable over a time period of less than a year, more preferably less than six months, most preferably over two to ten weeks. The polymer composition, as well as method of manufacture, can be used to determine the rate of degradation. For example, mixing increasing amounts of polylactic acid with polyglycolic acid decreases the degradation time. Meshes of polyglycolic acid that can be used can be obtained commercially, for instance, from surgical supply companies (e.g., Ethicon, N.J.). A hydrogel is defined as a substance formed when an organic polymer (natural or synthetic) is cross-linked via covalent, ionic, or hydrogen bonds to create a three-dimensional open-lattice structure which entraps water molecules to form a gel. In general, these polymers are at least partially soluble in aqueous solutions, such as water, buffered salt solutions, or aqueous alcohol solutions, that have charged side groups, or a monovalent ionic salt thereof.

For use in treatment of animal subjects, the compositions of the invention can be formulated as pharmaceutical or veterinary compositions. Depending on the subject to be treated, the mode of administration, and the type of treatment desired, e.g., prevention, prophylaxis, therapy; the compositions are formulated in ways consonant with these parameters. A summary of such techniques is found in Remington's Pharmaceutical Sciences, latest edition, Mack Publishing Co., Easton, Pa.

Preparation of therapeutic compositions which contain small organic molecules polypeptides, analogs or active fragments as active ingredients is well understood in the art. The compositions of the present invention may be administered parenterally, orally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, or intravenously. Formulations may be prepared in a manner suitable for systemic administration or for topical or local administration. Systemic formulations include, but are not limited to those designed for injection (e.g., intramuscular, intravenous or subcutaneous injection) or may be prepared for transdermal, transmucosal, nasal, or oral administration. Such compositions may be prepared as injectables, either as liquid solutions or suspensions, however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. The formulation will generally include a diluent as well as, in some cases, adjuvants, buffers, preservatives and the like. In addition, if desired, the composition can contain minor amounts of auxiliary substances such as wetting or emulsifying agents, pH buffering agents which enhance the effectiveness of the active ingredient.

A small organic molecule, a polypeptide, an analog or active fragment thereof can be formulated into the therapeutic composition as neutralized pharmaceutically acceptable salt forms. Pharmaceutically acceptable salts include the acid addition salts (formed with the free amino groups of the polypeptide or antibody molecule) and which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like. For oral administration, the compositions can be administered also in liposomal compositions or as microemulsions. Suitable forms include syrups, capsules, tablets, as is understood in the art.

The compositions of the present invention may also be administered locally to sites in subjects, both human and other vertebrates, such as domestic animals, rodents and livestock, using a variety of techniques known to those skilled in the art. For example, these may include sprays, lotions, gels or other vehicles such as alcohols, polyglycols, esters, oils and silicones.

Administration of the compositions of the present invention may be pharmacokinetically and pharmacodynamically controlled by calibrating various parameters of administration, including the frequency, dosage, duration mode and route of administration. Variations in the dosage, duration and mode of administration may also be manipulated to produce the activity required.

The therapeutic adenosine receptor modulator (e.g. inhibitor) compositions are conventionally administered in the form of a unit dose, for instance intravenously, as by injection of a unit dose, for example. The term "unit dose" when used in reference to a therapeutic composition of the present invention refers to physically discrete units suitable as unitary dosage for humans, each unit containing a predetermined quantity of active material calculated to produce the desired therapeutic effect in association with the required diluent; i.e., carrier, or vehicle.

The compositions are administered in a manner compatible with the agent selected for treating the subject, the dosage formulation, and in a therapeutically effective amount. If one desires to achieve the desired effect *in vitro,* the effective amounts may range from about 0:1 nM to about 10 µM, more preferably about 0.1 nM to about 5 µM, and most preferably from about 0.1 nM to about 1 nM. The desired effect refers to the effect of the agent on the liver, using the methods as described herein, and ultimately to an effect on amelioration of the symptoms associated with the hepatic disease or disorder, or a slowing of hepatic disease progression. Moreover, the quantity of the adenosine receptor antagonist to be administered depends on the subject to be treated, and degree of inhibition of the adenosine receptor desired or the extent or severity of hepatic disease. Precise amounts of active ingredient required to be administered depend on the judgment of the practitioner and are peculiar to each individual. However, suitable dosages to achieve the desired therapeutic effect *in vivo* may range from about 0.1 mg/kg body weight per day to about 200 mg/kg body weight per day, or from about 1.0 mg/kg body weight per day to about 100 mg/kg body weight per day, preferably about 25 mg/kg body weight per day to about 50 mg/kg body weight per day. The preferred dose will depend on the route of administration. However, dosage levels are highly dependent on the nature of the disease or situation, the condition of the subject, the judgment of the practitioner, and the frequency and mode of administration. If the oral route is employed, the absorption of the substance will be a factor effecting bioavailability. A low absorption will have the effect that in the gastro-intestinal tract higher concentrations, and thus higher dosages, will be necessary. Suitable regimes for initial administration and further administration are also variable, but are typified by an initial administration followed by repeated doses at one or more hour intervals by a subsequent injection or other administration. Alternatively, continuous intravenous infusion sufficient to maintain desired concentrations, e.g. in the blood, are contemplated. The composition may be administered as a single dose multiple doses or over an established period of time in an infusion.

Appropriate dosage of the substance should suitably be assessed by performing animal model tests, wherein the effective dose level (e.g. ED₅₀) and the toxic dose level (e.g. TD₅₀) as well as the lethal dose level (e.g. LD₅₀ or LD₁₀) are established in suitable and acceptable animal models. Further, if a substance has proven efficient in such animal tests, controlled clinical trials should be performed.

The compound or composition of the present invention may be modified or formulated for administration at the site of pathology. Such modification may include, for instance, formulation which facilitate or prolong the half-life of the compound or composition, particularly in the environment. Additionally, such modification may include the formulation of a compound or composition to include a targeting protein or sequence which facilitates or enhances the uptake of the compound/composition to bone or bone precursor cells. In a particular embodiment, such modification results in the preferential targeting of the compound to bone or bone precursor cells versus other locations or cells. In one embodiment, a tetracycline, tetracycline family or bisphosphonate may be utilized to target the compound or composition of the present invention to bone or bone cells, including osteoclasts and osteoclast precursors. Novel heterocycles as bone targeting compounds are disclosed in U.S. Patent Publication No. 2002/0103161 A1.

Pharmaceutically acceptable carriers useful in these pharmaceutical compositions include, e.g., ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Sterile injectable forms of the compositions of this invention may be an aqueous or an oleaginous suspension. These suspensions may be formulated according to techniques known in the art using suitable dispersing or wetting agents and suspending agents. The sterile injectable preparation may also be a sterile injectable solution or a suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Parenteral formulations may be a single bolus dose, an infusion or a loading bolus dose followed with a maintenance dose. These compositions may be administered once a day or on an "as needed" basis.

The pharmaceutical compositions of this invention may be orally administered in any orally acceptable dosage form including, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically. Topical application can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

### Effective Doses

Toxicity and therapeutic efficacy of compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds that exhibit large therapeutic indices are preferred. While compounds that exhibit toxic side effects can be used, care should be taken to design a delivery system that targets such compounds to the site of affected tissue in order to minimize potential damage to unaffected cells and, thereby, reduce side effects.

The data obtained from cell culture assays and animal studies can be used in formulating a dose range for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound which achieves a half-maximal inhibition of symptoms) as determined in cell culture. Such information can be used to optimize efficacious doses for administration to humans. Plasma levels can be measured by any technique known in the art, for example, by high performance liquid chromatography.

In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. Normal dose ranges used for particular therapeutic agents employed for specific diseases can be found in the Physicians' Desk Reference 54th Edition (2000).

### EXAMPLES

The following examples are provided to provide those of ordinary skill in the art with a complete disclosure and description of how to make and use the methods and compositions of the invention. Efforts have been made to insure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric. For examples 3 -7, the following detailed materials and methods were used as indicated.

Reagents. A₁ receptor agonist: N6-cyclopentyladenosine (CPA) (Jacobson, et al., Nat Rev Drug Discov (2006) 5:247-264), A₁ receptor antagonist: 1,3 dipropyl-8-cyclopentylxantine (DPCPX) (1), non-selective adenosine receptor agonist: 5'-N-ethylcarboxamidoadenosine (NECA) (Breschi et al., Eur J Pharmacol (2007) 575:149-157), A_{2B} antagonist: 3-propylxanthine (enprofylline) (Feoktistov et al., J Clin Invest (2005) 96:1979-1986; Fan et al., Am J Physiol Lung Cell Mol Physiol (2003) 284:L1012-1019) and A₃ antagonist: 3-ethyl-5-benzyl-2-methyl-4-phenylethynyl-6-phenyl-1,4-(±)-diphydropyridine-3,5-dicarboxylate (MRS1191) A₁ receptor agonist: N6-cyclopentyladenosine (CPA) (Jacobson, et al., Nat Rev Drug Discov (2006) 5:247-264), A₁ receptor antagonist: 1,3 dipropyl-8-cyclopentylxantine (DPCPX) (1), non-selective adenosine receptor agonist: 5'-N-ethylcarboxamidoadenosine (NECA) (Breschi et al., Eur J Pharmacol (2007) 575:149-157), A_{2B} antagonist: 3-propylxanthine (enprofylline) (Feoktistov et al., J Clin Invest (2005) 96:1979-1986; Fan et al., Am J Physiol Lung Cell Mol Physiol (2003) 284:L1012-1019) and A₃ antagonist: 3-ethyl-5-benzyl-2-methyl-4-phenylethynyl-6-phenyl-1,4-(±)-diphydropyridine-3,5-dicarboxylate (MRS1191) (5) were obtained from Sigma-Aldrich (St. Louis, MO). A more potent and selective A_{2B} receptor antagonist: N-(4-aceptylphenyl)-2-[4-(2,3,6,7-tetrahydro-2,6-dioxo-1,3-dipropyl-1H-purine-8-yl) phenoxoy]acetamide (MRS1706) (Breschi et al., Eur J Pharmacol (2007) 575:149-157; Kim et al., J Med Chem (2000) 43:1165-1172) and A_{2A} antagonist: 4-(2-[7-amino-2-(2-furyl)[1,2,4]triazolo[2,3-a][1,3,5]triazin-5-ylamino]ethyl)phenol (ZM 241385) (1) were purchased from Tocris Cookson (Avonmouth, UK). [³H]-DPCPX (specific activity 120 Ci/mmol) was obtained from Perkin Elmer Life and Analytical Sciences (Boston, MA, USA). [³H]-ZM 241385 (specific activity 17 Ci/mmol) was obtained from Tocris Cookson Ltd (Bristol, UK). N-benzo[1,3]dioxol-5-yl-2-[5-(1,3-dipropyl-2,6-dioxo-2,3,6,7-tetrahydro-1H-purin-8-yl)-1-methyl-1H-pyrazol-3-yloxy]-acetamide] ([3H]-MRE 2029F20, A_{2B} receptor antagonist, specific activity 123 Ci/mmol) and 5-N-(4-methoxyphenyl-carbamoyl)amino-8-propyl-2(2furyl)-pyrazolo-[4,3e]-1,2,4-triazolo [1,5-c] pyrimidine ([³H]-MRE 3008F20, A₃ receptor antagonist, specific activity 67 Ci/mmol) were derived from Amersham International Chemical Laboratories (Buckinghamshire, UK).

Receptor antagonists: N-(4-aceptylphenyl)-2-[4-(2,3,6,7-tetrahydro-2,6-dioxo-1,3-dipropyl-1H-purine-8-yl) phenoxoy]acetamide (MRS1706) (Breschi et al., Eur J Pharmacol (2007) 575:149-157; Kim et al., J Med Chem (2000) 43:1165-1172) and A_{2A} antagonist: 4-(2-[7-amino-2-(2-furyl)[1,2,4]triazolo[2,3-a][1,3,5]triazin-5-ylamino]ethyl)phenol (ZM 241385) (1) were purchased from Tocris Cookson (Avonmouth, UK). [³H]-DPCPX (specific activity 120 Ci/mmol) was obtained from Perkin Elmer Life and Analytical Sciences (Boston, MA, USA). [³H]-ZM 241385 (specific activity 17 Ci/mmol) was obtained from Tocris Cookson Ltd (Bristol, UK). N-benzo[1,3]dioxol-5-yl-2-[5-(1,3-dipropyl-2,6-dioxo-2,3,6,7-tetrahydro-1H-purin-8-yl)-1-methyl-1H-pyrazol-3-yloxy]-acetamide] ([³H]-MRE 2029F20, A_{2B} receptor antagonist, specific activity 123 Ci/mmol) and 5-N-(4-methoxyphenyl-carbamoyl)amino-8-propyl-2(2furyl)-pyrazolo-[4,3e]-1,2,4-triazolo [1,5-c] pyrimidine ([³H]-MRE 3008F20, A₃ receptor antagonist, specific activity 67 Ci/mmol) were derived from Amersham International Chemical Laboratories (Buckinghamshire, UK).

Animals. C57BL/6 (WT) mice were purchased from the Jackson Laboratory (Maine, USA). Ecto-5'-nucleotidase knockout mice (CD73KO, C57BL/6 background) were generated as described and provided as a gift by Dr. Linda Thompson (Oklahoma Medical Research Foundation) (Thompson et al., J Exp Med (2004) 200:1395-1405). A₁ knockout mice (A1KO) were a gift of Dr. Bertil Fredholm (Karolinska Institutet, Stockholm, Sweden) ( Johansson et al. Proc Natl Acad Sci U S A (2001) 98:9407-9412). CD73 knockout mice and A₁ receptor knockout mice were bred onto a C57BL/6 background (>10 backcrosses) in the NYU School of Medicine Animal Facility. Adenosine A_{2B} receptor knockout mice (A2BKO on a C57BL/6 background) have been previously described (Hua et al., J Exp Med (2007) 204:117-128). Adenosine receptor A_{2A} knockout mice (A2AKO, S129 mixed background) and their corresponding wild type littermates (Chen et al., J Neurosci (1999) 19:9192-9200) were bred in the NYU School of Medicine animal facility. All experimental mice were 6-8 week old male mice. All experimental procedures were approved by and performed in accordance with the guidelines of the Institutional Animal Care and Use Committee of the School of Medicine of New York University.

Mouse models of fatty livers and treatment. Mice were fed an ethanol-containing liquid Lieber-De-Carli diet (ethanol provides 350 Kcal/L) or a calorie equivalent diet supplemented with maltose (BioServ Inc., Frenchtown, NJ) for 6 weeks. A₁ receptor antagonist DPCPX (50mg/Kg/Day) or A_{2B} receptor antagonist enprofylline (50mg/Kg/Day) were administered to respective WT mice. In first week, animals were gradually introduced to the ethanol diet following the manufacturer's instructions. Mice were sacrificed by CO₂ narcosis at the end of the experiment and their livers were collected. Two wild type mice and one CD73 knockout mouse on an ethanol-containing diet died, and none of the antagonist-treated or other knockout mice died during the course of these experiments.

Hematoxylin-Eosin (H&E) and Oil Red O staining of liver sections. Livers were fixed with 10% neutral formalin, embedded in paraffin and stained with H&E for histological examination. Liver steatosis was graded as previously described (Hong et al., Hepatology (2004) 40:933-941) based on the semiquantitative estimation of the percentage of lipid-laden hepatocytes in the liver according to the following criteria: grade 0: no hepatocytes involved; grade 1: 1% to 25% of hepatocytes involved; grade 2: 26% to 50% of hepatocytes involved; grade 3: 51% to 75% of hepatocytes involved; and grade 4: 76% to 100% of hepatocytes involved. At least five random different high power fields (hpf) of each cross-section (400× magnification) were graded in a blinded way. For Oil Red O (Sigma-Aldrich, USA) staining, liver sections were flash-frozen and embedded in the frozen medium (optimal cutting temperature compound, Ted Pella, USA), 5-µm sections were cut and fixed to microscope slides, allowed to air dry overnight at room temperature and stained with fresh Oil Red O for 15 minutes, rinsed in water and then counterstained with hematoxylin.

Measurements of serum AST and triglyceride. Whole blood was taken from mice at the time of sacrifice and serum was isolated. AST and triglyceride were measured by standard techniques in the Clinical Laboratory of Bellevue Hospital.

Hepatic triglyceride measurement. Hepatic triglyceride measurements were made as previously described (Hong et al., Hepatology (2004) 40:933-941). In brief, liver tissue was homogenized at 4°C in RIPA lysis buffer (Sigma-Aldrich, USA). Lipids from the total liver homogenate were extracted using chloroform/methanol method (2:1), evaporated, and dissolved in 2-propanol. Triglyceride concentration was assayed enzymatically using kits obtained from Sigma-Aldrich (USA) following the manufacturers' instructions.

Hepatocyte cell culture, triglyceride measurement and oil red O staining of hepatocytes. The mouse hepatocyte cell line AML-12 was obtained from The American Type Culture Collection (Manassas, VA). These cells exhibit a differentiated and nontransformed hepatocyte phenotype, and have been previously used in numerous studies of hepatocyte injury (Schadinger et al., Am J Physiol Endocrinol Metab (2005) 288:E1195-1205; Sahai et al., Am J Physiol Gastrointest Liver Physiol (2006) 291:G55-62; Shen et al., Hepatology (2008) 47:473-483). AML-12 Cells were grown in 1:1 mixture of DMEM-Ham's F-12 medium supplemented with 0.005mg/ml insulin, 0.005mg/ml transferrin, 5ng/ml selenium, 40ng/ml dexamethasone, 10% fetal bovine serum and antibiotics. Upon reaching 70-80% confluence the cells were treated for 6 hours in serum-free DMEM-Ham's F-12 medium and then treated with different adenosine receptor agonists, antagonists or vehicle (DSMO, 1.28×10⁻⁵M), respectively. The method of the triglyceride measurement of hepatocytes is the same as the liver tissue. The cells were fixed with 10% neutral formalin, and Oil Red O staining as described above.

Real-time PCR quantitation of mRNA. Total RNA was isolated from liver tissue using Trizol reagent (Invitrogen, USA) and RNA extracted according to the manufacturer's instructions, then dissolved in sterile DEPC water and stored at -80° C. 1 µg of sample RNA was transcribed to cDNA with the GeneAmp RNA Core Kit (Applied Biosystems, Branchburg, NJ). Primer sequences for ATP citrate lyase (ACL), fatty acid synthase (FAS), sterol response element binding protein 1 (SREBP1), acetyl-CoA carboxylase (ACC), carnitine palmitoyltransferase (CPT), peroxisome proliferator-activated receptor (PPAR)α, PPAPγ and GAPDH are listed in Table I. PCR was performed with the SYBR Green PCR Kit (Applied Biosystems, Foster City, CA) following the manufacturer's instructions and carried out on the Mx3005P™ Q-PCR system (Stratagene, La Jolla, CA) in 50µl volume. The number of copies for each amplicon was calculated by Mx3005P software, normalized to GAPDH and expressed as a dimensionless ratio.

Western blotting and complete transcription factor measurement. Total cell protein extraction was carried out with RIPA lysis buffer (Sigma-Aldrich, USA), and tissue protein extraction was with T-PER tissue protein extraction reagent (Pierce, USA) after tissue being homogenized. The nuclear protein extractions were completed with NE-PER Nuclear and Cytoplasmic Extraction Reagents (Pierce, USA). Briefly, cells were collected and dissolved in the ice-cold cytoplasmic extraction reagent I, then cytoplasmic extraction reagent II was added to the cell pellet to remove cytoplasm, finally nuclear extraction reagent was added and nuclear protein was isolated. The extracting nuclear protein was used for western blot or transcription factor assay. ACL, FAS and phospho-AMPKa antibodies were purchased from Cell Signaling (USA), AMPKα, ACCα and CPTI antibodies were from Santa Cruz (USA), and SREBP-1, PPAR-α, PPAR-γ, β-actin and nuclear matrix protein p84 (P84) antibodies were from ABCAM (USA). 20 to 60µg proteins were loaded to 5% or 10% SDS-polyacrylamide gel and separated by electrophoresis, then transferred to nitrocellulose membranes. Western blot analyses were performed using the primary and second antibodies, those membranes were scanned on a phosphorImager (Amersham Biosciences), and the protein bands in the membranes were quantified using the Gel Logic 200 Image System (Kodak, USA) and normalized to β-actin or P84. The PPAR-α, PPAR-γ transcription factor assay was carried out by enzyme-linked immunosorbent assay (Elisa) following the manufacturer's instructions (Cayman Chemical, USA). This assay is a sensitive method for detecting specific transcription factor DNA binding activity in nuclear extracts, as compared with the traditional electrophoretic mobility shift assay (EMSA) (Li, et al., Assay Drug Dev Technol 4:333-341; Ogata et al., J Am Coll Cardiol (2004) 43:1481-1488). A specific double stranded DNA (dsDNA) sequence containing the peroxisome proliferator response element (PPRE) is immobilized onto the bottom of wells of a 96 well plate. PPARs contained in the nuclear extract bind specifically to the PPRE. PPAR-α and PPAR-γ are detected by addition of specific primary antibody directed against PPAR-α or PPAR-γ respectively. A second antibody conjugated to HRP is added. Absorbance was measured at 450 nm.

Preparation of human liver membranes. Fresh de-identified specimens of liver were obtained following abdominal surgery or liver transplant and maintained in a frozen tissue bank at Ferrara University by the Department of Surgery, Anesthesiology and Radiology and Department of Biomedical Sciences and Advanced Therapy. The normal control liver specimens were matched for age to the cohort of patients. Each sample was immediately frozen in liquid nitrogen and stored at -80° C until the assays were performed. The liver tissues were homogenized and filtered through two layers of gauze and the homogenate was centrifuged at 48,000 rpm for 30 min followed by resuspension of the pellet in a buffer containing 2 IU/ml of adenosine deaminase and incubated for 30 min at 37°C. The suspension was centrifuged again and the final pellet was used for radioligand binding assays.

[³H]-DPCPX, [³H]-ZM 241385, [³H]-MRE 2029F20 and [³H]-MRE3008F20 binding assays to human A₁, A_{2A}, A_{2B} and A₃ adenosine receptors respectively. Saturation binding experiments were performed as previously described using [³H]-DPCPX, [³H]-ZM 241385, [³H]-MRE 2029F20 and [³H]-MRE3008F20 as radioligands (Borea et al., Eur J Pharmacol (1994) 267:55-61; Varani, K. et al., Circulation (2000) 102:285-289; Gessi et al., Mol Pharmacol (2005) 67:2137-2147; Varani, K. et al., Mol Pharmacol (2000) 57:968-975). The membranes (60-100 µg of protein/assay) obtained from human healthy or diseased liver tissues were incubated with 8 to 10 concentrations of the radio ligand [³H]-DPCPX, [³H]-ZM 241385, [³H]-MRE 2029F20 and [³H]-MRE3008F20 (0.01-40 nM) for 60-150 min at 4°C-25°C. Non-specific binding was determined in the presence of excess unlabelled DPCPX, ZM 241385, MRE 2029F20 and MRE3008F20 (1 µM). Bound and free radioactivity was separated by filtering the assay mixture through Whatman GF/B glass fiber filters using a Brandel cell harvester. The filter bound radioactivity was counted using a Packard 2500 TR liquid scintillation counter with an efficiency of 58%. The protein concentration was determined by Bio-Rad method with bovine albumin as reference standard.

Statistical analysis. Data were expressed as means ± SD and were analyzed by Student's *t*-Test or ANOVA analysis, as appropriate, with SPSS software (SigmaStat) 10.0. We assigned statistical significance if *P* < 0.05.

### Example 1

### Adenosine Receptor A₁ Role in Fatty Liver

### Materials and methods

Adenosine receptor A₁ knockout mice (KO, S129 background) and wild type S129 (WT) mice were bred in the NYU Berg Animal Facility. All experimental mice were 6-8 week old male mice. All experimental procedures were approved by and performed in accordance with the guidelines of the Institutional Animal Care and Use Committee of School of Medicine of New York University. Mice were fed an ethanol-containing liquid Lieber-De-Carli diet (ethanol provide 350 Kcal/L) whereby ethanol was substituted isocalorically for maltose (BioServ Inc., Frenchtown, NJ) for 6 weeks. In the first week, ethanol animals were gradually introduced to the ethanol diet as per the manufacturers' instructions. Mice were sacrificed by CO₂ narcosis at the end of the experimental period (6 weeks) and their livers were collected. Hematoxylin-Eosin (H&E) and Oil Red O staining of liver sections were fixed with 10% neutral formalin, were sliced and stained with H&E for histological examination. Liver steatosis was graded blindly as previously described (Hong F, et al. Hepatology 2004; 40:933-941). Steatosis was semiquantitated based on the percentage of hepatocytes according to the following criteria: grade 0: no hepatocytes involved; grade 1: 1% to 25% of hepatocytes involved; grade 2: 26% to 50% of hepatocytes involved; grade 3: 51% to 75% of hepatocytes involved; and grade 4: 76% to 100% of hepatocytes involved. At least five different high power fields (hpf) of each cross-section (400× magnification) were graded in a blinded fashion. For Oil Red O (Sigma) staining, 5-µm sections were cut from flash frozen liver sections at a temperature of -80°C, the optimal cutting temperature samples, affixed to a microscope slide, and allowed to air dry overnight at room temperature. Liver sections were stained in fresh Oil Red O for 15 minutes, rinsed in water, and then counterstained with hematoxylin.

Whole blood was taken from mice at the time of sacrifice and the serum was isolated. AST, ALT, cholesterol and triglyceride were measured by standard techniques in the Clinical Laboratory of Bellevue Hospital. Hepatic triglyceride measurements were measured using the method of Hong (Hong F, et al. Hepatology 2004; 40:933-941). Briefly, livers were homogenized at 4°C in lysis buffer containing 50 mmol/L, 150 mmol/L NaCl, 1% Triton X-100, and 0.5% Na DOC. Lipids from the total liver homogenate were extracted using chloroform/methanol method (2:1), evaporated, and dissolved in 2-propanol. Amounts of triglyceride were assayed enzymatically using kits obtained from Sigma Co. (USA) and the results were expressed per mg of hepatic tissue.

A mouse hepatocyte cell line AML-12 was obtained from The American Type Culture Collection (Manassas, VA). These cells exhibit differentiated, nontransformed hepatocyte phenotype and have been previously used in numerous studies of hepatocyte injury. AML-12 Cells were grown in 1:1 mixture of DMEM-Ham's F-12 medium supplemented with 10% fetal bovine serum and antibiotics. Cells reached 70-80% confluence, cultured for 6 hours in serum-free DMEM-Ham's F-12 medium and then treated with the adenosine A₁ receptor agonist N⁶-cyclopentyladenosine (CPA, 10⁻⁶M), the adenosine A₁ receptor antagonist 8-cyclopenthyldipropylxanthine (DPCPX,10⁻⁶M) or vehicle containing DSMO (<0.01%) for 24 hours.

The cells were fixed with 10% neutral formalin, and Oil Red O staining carried out, as described above. Total RNA was isolated from cultured hepatocytes using Trizol reagent (Invitrogen, USA) and RNA extracted according to the manufacturer's instructions, then dissolved in sterile DEPC water and stored at -80°C. 1 µg RNA of sample RNA was transcribed to cDNA with the GeneAmp RNA Core Kit (Applied Biosystems, Branchburg, NJ). Primer sequences ATP citrate lyase (ACL): forward 5'-GAA GCT GAC CTT GCT GAA CC-3', reverse 5'-CTG CCT CCA ATG ATG AGG AT-3'; fatty acid synthase (FAS): forward 5'-TGG GTT CTA GCC AGC AGA GT-3', reverse: 5'-ACC ACC AGA GAC CGT TAT GC-3'. PCR was performed with the SYBR Green PCR Kit (Applied Biosystems, Foster City, CA) following the manufacturer's instructions and carried out on the Mx3005P™ Q-PCR system (Stratagene, La Jolla, CA) in 50µl volume. The number of copies for each amplicon was calculated by Mx3005P software, normalized to GAPDH and expressed as a dimensionless ratio.

### Results.

Data were expressed as means ± SD and were analyzed by Student's *t*-Test or ANOVA analysis, as appropriate, with SPSS software 10.0. We assigned statistical significance if *P* < 0.05.

After sacrifice of the mice liver weight was measured, and liver/body weight ratios were calculated. The liver/body weight ratio had significantly more increase in ethanol, fatty diet-treated-mice compared with control mice (P<0.01), but no difference was observed between WT and KO mice either after a normal mouse diet or following 6 weeks of a high fat, ethanol-containing diet. (FIG. 1).

Severe hepatic steatosis was observed in WT mice following ethanol treatment for 6 weeks, as demonstrated by both H&E staining and Oil Red O staining. Liver sections from A. WT mouse treated with normal diet (H&E stain); B. A₁ receptor KO mouse treated with normal diet (H&E Stain); C. WT mouse treated with high fat/ethanol containing diet (H&E stain); D. A₁ knockout mouse treated with high fat/ethanol containing diet (H&E Stain); E. WT mouse treated with high fat/ethanol containing diet (Oil Red O stain); F. A₁ knockout mouse treated with high fat/ethanol containing diet (Oil Red O Stain). (FIG. 2).

Serum AST, ALT and triglyceride were significantly higher in WT mice than these in KO mice following high fat/ethanol treatment for 6 weeks (AST: 232.0 ± 30.9 UI/L vesus 103.3 ± 15.5 UI/L; ALT: 69.5 ± 5.97 UI/L versus 38.3 ± 5.1 UI/L; 92.5 ± 9.2 mg/dlversus 49.3 ± 9.4 mg/dl; n=6 in each group, *P*<0.01 respectively). (FIG. 3A) Hepatic triglyceride was increased in high fat/ethanol-treated mice compared to control diet-treated mice, but there was much greater increase in WT than KO mice following treatment with high fat/ethanol diet animals (90.6 ± 11.0 mg/g versus 66.8 ± 10.5 mg/g, n=6 in each group, P<0.01). (FIG. 3B) Hepatic steatosis score was significantly higher in WT mice than in KO mice treated with high fat/ethanol diet (3.83 ± 0.39 versus 2.08 ± 0.32, n=6 in each group, P<0.01). (FIG. 3C).

Adenosine receptor A₁ agonist CPA significantly increased the lipid content of hepatocytes and its effect was nearly completely blocked by the adenosine A₁ receptor antagonist DCPX. (FIG. 4)

mRNA for ACL and FAS were significantly increased following CPA treatment for 24 hours and this increase was completely blocked by DPCPX (ACL: control 100.0%, CPA 181.4 ± 12.9%, DCPX 92.0 ± 5.2%, CPA+DCPX 110.0 ± 7.5%; FAS: control 100.0%, CPA 164.2 ± 6.8%, DCPX 105.8 ± 5.3%, CPA+DCPX 108.3 ± 5.6%, n=3 in each group, CPA versus any other group, P<0.01 respectively). (FIG. 5)

### Example 2

### Adenosine Receptor A_{2B} Role in Fatty Liver

### Materials and methods

Wild-type mice were purchased from the Jackson Laboratory (Maine, USA). Adenosine receptor A_{2B} knockout mice (A_{2B} KO, C57BL/6 background) mice were obtained from Dr. Blackburn's laboratory at the University of Texas at Houston. Animals were bred in the animal facilities of the School of Medicine of the New York University. All experimental mice were 6-8 week old male mice. All experimental procedures were approved by and performed in accordance with the guidelines of the Institutional Animal Care and Use Committee of School of Medicine of New York University.

Mice were fed an ethanol-containing liquid Lieber-De-Carli diet (ethanol provided 350 Kcal/L) whereby ethanol was substituted isocalorically which maltose (BioServ Inc., Frenchtown, NJ) for 6 weeks. In the first week, ethanol animals were gradually introduced to the ethanol diet following factory instruction. Mice were sacrificed by CO₂ narcosis at the end of the experiment and their livers were collected.

Livers were fixed with 10% neutral formalin and were sliced and stained with H&E for histological examination. Liver steatosis was graded according to the guidelines of Hong. (Hong F, et al. Hepatology 2004; 40:933-941). Steatosis was semiquantitated based on the percentage of hepatocytes according to the following criteria: grade 0: no hepatocytes involved; grade 1: 1% to 25% of hepatocytes involved; grade 2: 26% to 50% of hepatocytes involved; grade 3: 51% to 75% of hepatocytes involved; and grade 4: 76% to 100% of hepatocytes involved. At least five random different high power fields (hpf) of each cross-section (400× magnification) were graded in a blind way.

Whole blood was taken from mice at the time of sacrifice, and the serum was isolated. AST, ALT, cholesterol and triglyceride were measured by standard techniques in the Clinical Laboratory of Bellevue Hospital. Hepatic triglyceride measurement was performed following the methods described by Hong (Hong F, et al. Hepatology 2004; 40:933-941). Livers were homogenized at 4°C in lysis buffer containing 50 mmol/L, 150 mmol/L NaCl, 1% Triton X-100, and 0.5% Na DOC. Lipids from the total liver homogenate were extracted using chloroform/methanol method (2:1), evaporated, and dissolved in 2-propanol. Amounts of triglyceride were assayed enzymatically using kits obtained from Sigma Co. (USA).

### Results.

Data were expressed as mean ± SD and were analyzed by a Student's *t*-Test or ANOVA analysis, as appropriate, with SPSS software 10.0. We assigned statistical significance if *P* < 0.05. Less liver weight, triglyceride and steatosis grade was present in ethanol-treated A_{2B} mice. (FIG. 6A) At the end of experiment, liver weight was measured, and liver/body weight ratios were calculated. The liver/body weight ratio (%) was much less in ethanol treated A_{2B} KO mice compared with wild-type mice (4.46 ± 0.25% versus 5.09 ± 0.19%, n=4 in each group, *P*<0.01). Serum triglyceride levels were significantly higher in WT mice than serum triglyceride levels in A_{2B} KO mice following ethanol treatment for 6 weeks (154.83 ± 30.56 mg/dl versus 82.0 ± 20.27 mg/dl; n=4 in each group, *P*<0.01). (FIG. 6B) Hepatic triglyceride lever were also more increased in WT mice than that in A_{2B} KO mice in ethanol-treated animals (92.98 ± 12.34 mg/g versus 64.41 ± 5.10 mg/g, n=4 in each group, *P*<0.01). (FIG. 6C) The hepatic steatosis score was much higher in WT mice than that in A_{2B} KO mice in ethanol-treated animals (4.33 ± 0.39 versus 2.10 ± 0.41, n=4 in each group, P<0.01). (FIG. 6D). Severe hepatic steatosis was observed in WT mice following ethanol treatment for 6 weeks in H&E staining. (FIG. 7)

### Example 3

### Deletion of ecto-5'Nucleotidase (CD73) prevents the development of ethanol-induced fatty liver in mice

Materials and Methods. Eight-week old male mice (wild type and CD73KO mice) were fed a liquid diet containing ethanol (350Kcal/L) or an equal caloric diet containing maltose for six weeks. Mice were then sacrificed at the end of six weeks, their livers were collected and stained with H&E and Oil-Red O staining. The liver and body of the mice were weighed on the day of sacrifice and the liver/total body weight ratio calculated. The serum AST and triglyceride and hepatic tissue triglyceride levels were also measured.

Measurements of serum AST and triglyceride. Whole blood was taken from mice at the time of sacrifice and serum was isolated. AST and triglyceride were measured by standard techniques in the Clinical Laboratory of Bellevue Hospital.

Hepatic triglyceride measurement. Hepatic triglyceride measurements were made as previously described (Hong et al., Hepatology (2004) 40:933-941). In brief, liver tissue was homogenized at 4°C in RIPA lysis buffer (Sigma-Aldrich, USA). Lipids from the total liver homogenate were extracted using chloroform/methanol method (2:1), evaporated, and dissolved in 2-propanol. Triglyceride concentration was assayed enzymatically using kits obtained from Sigma-Aldrich (USA) following the manufacturers' instructions.

Hepatocyte cell culture, triglyceride measurement and oil red O staining of hepatocytes. The mouse hepatocyte cell line AML-12 was obtained from The American Type Culture Collection (Manassas, VA). These cells exhibit a differentiated and nontransformed hepatocyte phenotype, and have been previously used in numerous studies of hepatocyte injury (Schadinger et al., Am J Physiol Endocrinol Metab (2005) 288:E1195-1205; Sahai et al., Am J Physiol Gastrointest Liver Physiol (2006) 291:G55-62; Shen et al., Hepatology (2008) 47:473-483). AML-12 Cells were grown in 1:1 mixture of DMEM-Ham's F-12 medium supplemented with 0.005mg/ml insulin, 0.005mg/ml transferrin, 5ng/ml selenium, 40ng/ml dexamethasone, 10% fetal bovine serum and antibiotics. Upon reaching 70-80% confluence the cells were treated for 6 hours in serum-free DMEM-Ham's F-12 medium and then treated with different adenosine receptor agonists, antagonists or vehicle (DSMO, 1.28×10⁻⁵M), respectively. The method of the triglyceride measurement of hepatocytes is the same as the liver tissue. The cells were fixed with 10% neutral formalin, and Oil Red O staining as described above.

Results. The hepatic steatosis grade was based on the percentage of steatotic hepatocytes in the H&E stained liver sections. Figure 8A provides H&E stained liver sections from maltose and ethanol-treated WT and CD73KO mice (Original magnification ×400). Figure 8B demonstrates hepatic steatosis grades of ethanol-treated WT and CD73KO mice (Original magnification ×400). Steatosis grades in maltose-treated WT and CD73KO mice were 0. Figure 8C demonstrates Oil Red O stained liver sections (Original magnification x400) from ethanol-treated WT and CD73KO mice. Figure 8D demonstrates liver/body ratio of CD73KO and WT mice. Figure 8E demonstrates serum AST levels of WT and CD73KO mice. Figure 8F demonstrates serum triglyceride levels of WT and CD73KO mice. Figure 8G demonstrates hepatic tissue triglyceride levels in WT and CD73KO mice. (#CD73KO mice versus WT mice, *P*<0.01; *ethanol mice versus control mice in different groups, respectively, *P*<0.01; **ethanol CD73KO mice versus control WT mice or ethanol WT mice, *P*<0.01, respectively. N=5-10 for each group of mice.)

Discussion. Livers from mice that have ingested ethanol release more adenosine *ex vivo* than livers from non-ethanol exposed mice and the increase in adenosine release is largely dependent on extracellular dephosphorylation of AMP to adenosine by CD73 since deletion of this enzyme leads to markedly diminished adenosine release (Peng et al, Faseb J (2008) 22:2263-2272). To determine whether ethanol-induced, CD73-dependent adenosine accumulation might play a role in the development of ethanol-induced hepatic steatosis, the effect of chronic ethanol ingestion on the liver in CD73KO and wild type mice was studied. Wild type mice developed severe hepatic steatosis after chronic ethanol ingestion with obvious lipid droplets present in nearly 100% of hepatocytes in both periportal and pericentral areas but CD73KO mice suffered only minimal fatty change (Figure 8A, B). Moreover, Oil Red O staining showed a marked reduction in the large intracellular lipid droplets in the livers of CD73KO mice as compared to WT mice (Figure 8C). Consistent with the histological appearance, the triglyceride content of hepatic tissue was much lower in ethanol-fed CD73KO mice than in wild type mice although, consistent with the histologic findings, hepatic triglyceride levels in CD73KO mice were slightly higher than in non-ethanol fed mice (Figure 8G). Serum AST levels reflect ethanol-induced hepatic injury and serum triglyceride levels are elevated in animals or patients with ethanol-induced fatty liver; serum AST levels and triglycerides were significantly lower in CD73KO mice than in WT mice as well (Figure 8E, F). Thus, deletion of CD73 and reduction in ethanol-induced hepatic adenosine production protects mice from the development of ethanol-induced fatty liver.

### Example 4

### Deletion of adenosine A₁ or A_{2B} receptors protects mice from developing ethanol-induced fatty liver

Materials and Methods. Eight-week old male mice (wild type, A1KO and A2BKO mice, and A1WT and A1KO mice) were fed a liquid diet containing ethanol (350Kcal/L) or an equal caloric diet containing maltose for six weeks and then sacrificed. The liver and body of the mice were weighed on the day of sacrifice and the liver/total body weight ratio calculated. The serum AST and triglyceride and hepatic tissue triglyceride levels were also measured as described in Example 3, *supra.* The hepatic steatosis grade was based on the percentage of steatotic hepatocytes in the H&E stained liver sections.

Results. Figure 9A demonstrates H&E stained liver sections from maltose and ethanol-treated A1KO, A2BKO, A2AKO and WT mice (Original magnification × 400). Figure 9B demonstrates H&E stained liver sections from maltose and ethanol- treated A2AKO and WT mice (Original magnification × 400). Figure 9B demonstrates Oil Red O stained liver sections (Original magnification x 400) from ethanol-treated A1KO and A2BKO mice. Figure 9C demonstrates hepatic steatosis grades of ethanol-treated A1KO, A2BKO, A2AKO and WT mice (Original magnification × 400). Steatosis grades in maltose-treated A1KO, A2BKO and A2AKO mice were 0. Figure 9D demonstrates Liver/body ratio of A1KO, A2BKO, A2AKO and WT mice. Figure 9E demonstrates serum AST levels of A1KO, A2BKO and WT mice. Figure 9F demonstrates serum triglyceride levels of A1KO, A2BKO, A2AKO and WT mice. Figure 9G demonstrates hepatic tissue triglyceride levels in A1KO, A2BKO,A2AKO and WT mice. (#A1KO mice or A2BKO mice versus WT mice, *P*<0.01, respectively; *ethanol mice versus control mice in different groups, respectively, *P*<0.01; **ethanol KO mice versus control WT mice or ethanol WT mice, *P*<0.01, respectively. N=5-10.)

Discussion. Adenosine and its receptors play a role in the pharmacologic effects of ethanol. (Diamond et al., Annals of the New York Academy of Sciences (1991) 625:473-487; Gordon et al., Biochemical Society Symposia (1990) 56:117-136; Nagy et al., Molecular Pharmacology (1989) 36:744-748; Anwer et al., Pharmacology (1995) 51:364-369; Meng et al., Alcoholism, Clinical & Experimental Research (1995) 19:892-901; Sapru *et al.,* Journal of Pharmacology & Experimental Therapeutics (1994) 271:542-548; Dar et al., Alcohol & Alcoholism. Supplement (1993) 2:425-429; Carmichael et al., Alcohol & Alcoholism. Supplement (1993) 2:411-418; Connole et al., Basic Clin Pharmacol Toxicol (2004) 95:299-304; El Yacoubi et al., Neuropharmacology (2003) 45:977-985; Dar et al., Brain Res (2002) 957:53-60; Campisi et al., Eur J Pharmacol (1997) 325:165-172; Israel et al., Alcohol Clin Exp Res (1994) 18:144-148; Orrego et al., Am J Physiol (1988) 254:G495-501; Biggs et al., Alcohol (1997) 14:617-621; Malec et al., Pol JPharmacol (1996) 48:583-588; Dar et al., Alcohol Clin Exp Res (1992)16:1138-1146; Carmichael et al., Am J Physiol (1988) 255:G417-423; Proctor et al., Science (1984) 224:519-521; 21. Dar et al., Life Sci (1983) 33:1363-1374). To determine whether and which adenosine receptors play a role in the pathogenesis of fatty liver we studied the ethanol-induced development of hepatic steatosis in mice lacking adenosine A₁, A_{2A} and A_{2B} receptors. As shown in Figure 9A, adenosine A₁ and A_{2B} but not A_{2A} receptor deletion protects mice from the development of hepatic steatosis following ethanol ingestion. Fewer lipid-laden hepatocytes were observed in the H&E-stained hepatic sections of A₁KO and A_{2B}KO mice but not A_{2A}KO, mice as compared to wild type control mice and Oil Red O staining of frozen sections of the liver confirms the reduction in hepatic steatosis in A₁KO and A_{2B}KO mice (Figure 9A, B). Interestingly, the liver/total body weight ratio did not differ between ethanol-treated WT mice and A₁KO or A_{2B}KO mice (Figure 9D). Hepatic triglyceride content and steatosis grade was less in the A₁KO and A_{2B}KO mice but not the A_{2A}KO mice (Figure 9C, G) and serum triglycerides and AST were significantly reduced in the A₁KO and A_{2B}KO, but not the A_{2A}KO mice as well (Figure 9E, F).

### Example 5

### Blockade of adenosine A₁ or A_{2B} receptors protects mice from developing ethanol-induced fatty liver

Materials and Methods. Eight week old C57B1/6 male mice were fed a liquid diet containing ethanol (350 Kcal/L) or an equal caloric diet containing maltose for six weeks supplemented with A₁ receptor-specific antagonist DPCPX, the A2 B receptor-specific antagonist enprofylline or vehicle and then sacrificed. The liver and body of the mice were weighed on the day of sacrifice and the liver/total body weight ratio calculated. The serum AST and triglyceride and hepatic tissue triglyceride levels were also measured, as described in Example 3, *supra.* The hepatic steatosis grade was based on the percentage of steatotic hepatocytes in the H&E stained liver sections. Figure 10A demonstrates H&E and Oil Red O staining of liver sections of DPCPX- or enprofylline-treated mice (Original magnification × 400). Figure 10B demonstrates hepatic steatosis grades. Figure 10C demonstrates liver/ body ratio. Figure 10D demonstrates serum AST levels. Figure 10E serum triglyceride levels. Figure 10F hepatic tissue triglyceride levels. (*Drug-treated ethanol mice vs ethanol mice in different groups, P<0.01, respectively; **drug-treated ethanol mice vs control mice in different groups, *P*<0.01 or P<0.05, respectively. N=5-10 in each group.)

Results. Serum triglycerides and AST were elevated in the ethanol-treated wild type mice. AST levels were significantly lower in both enprofylline (A_{2B} antagonist)- and DPCPX (A₁ antagonist)-treated mice although there was only a significant reduction in serum triglyceride levels in the enprofylline-treated but not the DPCPX-treated mice (Figure 10D, E). Similar to the knockout mice, there were significant reductions in hepatic triglyceride levels in the antagonist-treated mice compared with the vehicle-treated mice although the levels did not return to those of mice not exposed to ethanol (Figure 10F). As with the hepatic triglyceride level, the hepatic steatosis grade was significantly decreased in all of the antagonist-treated mice compared with vehicle-treated mice (Figure 10B). Interestingly, the reduction in steatosis grade was greater in the receptor knockout and antagonist-treated mice than in ethanol-treated CD73KO mice (Figure 8B and Figure 10B). Thus, these results are consistent with the hypothesis that adenosine, acting through A₁ and A_{2B} receptors, plays a central role in the pathogenesis of ethanol-induced fatty liver.

### Example 6

### Ethanol ingestion modulates expression of hepatic transcription factors SREBP1 and PPARα and hepatic enzymes and transporters involved in fatty acid synthesis, metabolism and transport

Materials and methods. mRNA expression of genes in hepatic tissue was assessed by Real-time PCR and normalized to GAPDH. Total RNA was isolated from liver tissue using Trizol reagent (Invitrogen, USA) and RNA extracted according to the manufacturer's instructions, then dissolved in sterile DEPC water and stored at -80° C. 1 µg of sample RNA was transcribed to cDNA with the GeneAmp RNA Core Kit (Applied Biosystems, Branchburg, NJ). Primer sequences for ATP citrate lyase (ACL), fatty acid synthase (FAS), sterol response element binding protein 1 (SREBP1), acetyl-CoA carboxylase (ACC), carnitine palmitoyltransferase (CPT), peroxisome proliferator-activated receptor (PPAR)α, PPAPγ and GAPDH are listed in Table I. PCR was performed with the SYBR Green PCR Kit (Applied Biosystems, Foster City, CA) following the manufacturer's instructions and carried out on the Mx3005P™ Q-PCR system (Stratagene, La Jolla, CA) in 50µl volume. The number of copies for each amplicon was calculated by Mx3005P software, normalized to GAPDH and expressed as a dimensionless ratio.

Results. Figure 11A demonstrates mRNA expression of ACL. Figure 11B demonstrates mRNA expression of FAS. Figure 11C demonstrates mRNA expression of ACCα. Figure 11D demonstrates mRNA expression of CPTI. Figure 11E demonstrates mRNA expression of PPARα. Figure 11F demonstrates mRNA expression of PPARγ. Figure 11G demonstrates mRNA expression of SREBP1. (*Ethanol vs control in different groups, P<0.01, respectively; **DPCPX or enprofylline-treated mice vs ethanol WT mice, P<0.01, respectively, n=5 in each group)

Discussion. Increased or decreased expression/function of specific transcriptional regulators has previously been shown to play a role in the development of hepatic steatosis; SREBP1 increases expression of genes/proteins involved in lipid synthesis (including ATP Citrate lyase (ACL) and fatty acid synthase (FAS)) (Brown et al., Proc Natl Acad Sci U S A (1999) 96:11041-11048; Shimano et al., J Clin Invest (1997) 99:846-854; Osborne et al., J Biol Chem (2000) 275:32379-32382), PPARα regulates proteins involved in fatty acid oxidation (e.g. Acetyl CoA oxidase (ACCα) and carnitine palmitoyl transferase) (Aoyama et al., J Biol Chem (1998) 273:5678-5684; Kersten et al., J Clin Invest (1999) 103:1489-1498; Leone et al., Proc Natl Acad Sci USA (1999) 96:7473-7478), and PPAPγ mainly regulates lipogenesis (Tontonoz et al., Cell (1994) 79:1147-1156; Gavrilova et al., J Biol Chem (2003) 278:34268-34276; Yu et al., J Biol Chem (2003) 278:498-505; Schadinger et al., Am J Physiol Endocrinol Metab (2005) 288:E1195-1205). In order to determine whether adenosine and its receptors regulate the development of hepatic steatosis by altering the expression of molecules in these pathways we measured the mRNA expression of ACL, FAS, ACCα, CPTI, PPARα, PPAPγ and SREBP1 genes in mouse liver tissue by real-time PCR. Chronic ethanol ingestion increased mRNA expression of ACL, FAS, PPARγ and SREBP1 in wild type, A_{2B}KO and enprofylline-treated mice but adenosine A₁ receptor deletion or blockade (DPCPX treatment) prevented the ethanol-induced increase in expression (Figure 11A, B, F, G). In contrast, ethanol ingestion reduced mRNA expression of ACCα, CPTI and PPARα in the livers of wild type, A₁KO and DPCPX-treated but deletion or blockade (enprofylline treatment) of A_{2B} receptors abrogated the ethanol-induced reduction in expression (Figure 11A, C, D, E).

### Example 7

### Agonists of adenosine A₁ and A_{2B} receptors promote fat accumulation in a cultured murine hepatocyte cell line

Materials and methods. Cells were treated with adenosine receptor agonists or antagonists or their combination (A₁agonist, CPA1mM; A₁antagonist, DPCPX 1mM; non-selective and A2Bagonist, NECA 10mM; A_{2B} antagonist, MRS1706 1mM) for twenty-four hours, then stained with Oil Red O or collected and the cellular triglyceride measured.

Results. Figure 12A demonstrates Oil red O staining of AML-12 hepatocytic cells (Original magnification ×400). Figure 12B demonstrates cellular triglyceride content of AML-12 cells. The data are expressed as percentages of control (mean ±SD) from four independent experiments (*Treatment versus control, *P*<0.01; **antagonist or agonist + antagonist versus agonist, *P*<0.01, respectively).

Discussion. To better understand how adenosine A₁ and A_{2B} receptors are involved in the formation of fatty liver *in vivo,* we examined the *in vitro* effect of selective adenosine A₁ and A_{2B} receptor agonists and antagonists on development of steatosis in an hepatocyte cell line (AML-12). AML-12 cells express mRNA for all four adenosine receptors (data not shown). Following treatment with the A₁ receptor agonist CPA or the non-selective adenosine receptor agonist NECA at a concentration which activates A_{2B} receptors, AML-12 cells accumulated lipid, as demonstrated by Oil Red O staining, and increased their intracellular triglyceride levels (Figure 12A, D, E). The intracellular triglyceride content increased in response to both CPA and NECA in a dose-dependent fashion (Figure 12B, C). Addition of the adenosine A₁ or A_{2B} selective antagonists DPCPX or MRS 1706, respectively, almost completely abrogated the effects of the A₁ and A_{2B} receptor agonists on hepatocyte triglyceride accumulation, respectively, whereas DPCPX partly blocked the effect of NECA, and neither A_{2A} nor A₃ adenosine receptor antagonists blocked the effects of either CPA or NECA on hepatocyte triglyceride accumulation (Figure 12A, D, E).

## Claims

1. An A₁ or an A_{2B} adenosine receptor antagonist, or combination thereof, for use in a method of decreasing fat deposition in the liver.

2. The antagonist for use of claim 1, wherein the adenosine receptor antagonist is a selective adenosine receptor antagonist.

3. The antagonist for use of claim 1, wherein the adenosine receptor antagonist is selected from the group consisting of a small organic molecule, a protein or peptide, a nucleic acid and an antibody.

4. The antagonist for use of claim 1, wherein the adenosine A₁ receptor antagonist is selected from the group consisting of DPCPX, CPX, N-0861, N-0840, N-0861, CVT-124, WRC-0342, CGS-15943, XAC, WRC-0571, KW-3902, ENX, KFM 19 (BIIP20), FK453, FK352, FK838, FR166124, 8-cyclopentyltheophylline, BG9719 and BG9928.

5. The antagonist for use of claim 1, wherein the adenosine A₁ or A_{2B} receptor antagonist is administered orally or parenterally.

6. The antagonist for use of claim 1, wherein the adenosine A₁ or A_{2B} receptor antagonist is administered intravenously, subcutaneously, intramuscularly, or intravasally.

7. The antagonist for use of claim 1, wherein the adenosine A₁ or A_{2B} receptor antagonist is administered via an implanted device, or is administered to a mucous membrane.

8. The antagonist for use of claim 1, wherein the adenosine receptor antagonist is administered in combination with a therapeutically effective amount of one or more other compounds or agents effective for treating a hepatic disease or condition or for reducing fat deposition in the liver or fibrosis of the liver.

9. A pharmaceutical composition for use in a method of decreasing fat deposition in the liver according to claim 1, comprising an adenosine receptor antagonist selected from the group consisting of an A₁ and A_{2B} adenosine receptor antagonist and a pharmaceutically acceptable carrier.

## Patentansprüche

1. A₁- oder A_{2B}-Adenosinrezeptor-Antagonist oder eine Kombination daraus zur Verwendung in einem Verfahren zum Vermindern der Fettablagerung in der Leber.

2. Antagonist zur Verwendung nach Anspruch 1, wobei der Adenosinrezeptor-Antagonist ein selektiver Adenosinrezeptor-Antagonist ist.

3. Antagonist zur Verwendung nach Anspruch 1, wobei der Adenosinrezeptor-Antagonist ausgewählt ist aus der Gruppe bestehend aus einem kleinen organischen Molekül, einem Protein oder Peptid, einer Nukleinsäure und einem Antikörper.

4. Antagonist zur Verwendung nach Anspruch 1, wobei der Adenosin-A₁-Rezeptor-Antagonist ausgewählt ist aus der Gruppe bestehend aus DPCPX, CPX, N-0861, N-0840, N-0861, CVT-124, WRC-0342, CGS-15943, XAC, WRC-0571, KW-3902, ENX, KFM 19 (BIIP20), FK453, FK352, FK838, FR166124, 8-Cyclopentyltheophyllin, BG9719 und BG9928.

5. Antagonist zur Verwendung nach Anspruch 1, wobei der Adenosin-A₁- oder A_{2B}-Rezeptor-Antagonist oral oder parenteral verabreicht wird.

6. Antagonist zur Verwendung nach Anspruch 1, wobei der Adenosin-A₁- oder A_{2B}-Rezeptor-Antagonist intravenös, subkutan, intramuskulär oder intravasal verabreicht wird.

7. Antagonist zur Verwendung nach Anspruch 1, wobei der Adenosin-A₁- oder A_{2B}-Rezeptor-Antagonist über eine implantierte Vorrichtung verabreicht wird oder an eine Schleimhaut verabreicht wird.

8. Antagonist zur Verwendung nach Anspruch 1, wobei der Adenosinrezeptor-Antagonist in Kombination mit einer therapeutisch wirksamen Menge einer oder mehrerer Verbindungen oder Wirkstoffe verabreicht wird, die wirksam zum Behandeln einer Lebererkrankung oder eines Leberzustands oder zum Reduzieren einer Fettablagerung in der Leber oder einer Fibrose der Leber sind.

9. Pharmazeutische Zusammensetzung zur Verwendung in einem Verfahren zum Vermindern der Fettablagerung in der Leber gemäß Anspruch 1, umfassend einen Adenosinrezeptor-Antagonist ausgewählt aus der Gruppe bestehend aus einem A₁- und A_{2B}-Adenosinrezeptor-Antagonist und einem pharmazeutisch verträglichen Träger.

## Revendications

1. Antagoniste d'un récepteur A₁ ou A_{2B} de l'adénosine, ou de l'une de leurs combinaisons, pour une utilisation dans un procédé de diminution du dépôt de graisse dans le foie.

2. Antagoniste pour une utilisation selon la revendication 1, dans lequel l'antagoniste d'un récepteur de l'adénosine est un antagoniste sélectif d'un récepteur de l'adénosine.

3. Antagoniste pour une utilisation selon la revendication 1, dans lequel l'antagoniste d'un récepteur de l'adénosine est choisi dans le groupe constitué d'une petite molécule organique, une protéine ou un peptide, un acide nucléique et un anticorps.

4. Antagoniste pour une utilisation selon la revendication 1, dans lequel l'antagoniste d'un récepteur A₁ de l'adénosine est choisi dans le groupe constitué de DPCPX, CPX, N-0861, N-0840, N-0861, CVT-124, WRC-0342, CGS-15943, XAC, WRC-0571, KW-3902, ENX, KFM 19 (BIIP20), FK453, FK352, FK838, FR166124 et ses analogues, 8-cyclopentylthéophylline, BG9719 et BG9928.

5. Antagoniste pour une utilisation selon la revendication 1, dans lequel l'antagoniste d'un récepteur A₁ ou A_{2B} de l'adénosine est administré par voie orale ou parentérale.

6. Antagoniste pour une utilisation selon la revendication 1, dans lequel l'antagoniste d'un récepteur A₁ ou A_{2B} de l'adénosine est administré par voie intraveineuse, sous-cutanée, intramusculaire, ou intravasculaire.

7. Antagoniste pour une utilisation selon la revendication 1, dans lequel l'antagoniste d'un récepteur A₁ ou A_{2B} de l'adénosine est administré par l'intermédiaire d'un dispositif implanté, ou est administré à une membrane muqueuse.

8. Antagoniste pour une utilisation selon la revendication 1, dans lequel l'antagoniste d'un récepteur de l'adénosine est administré en combinaison avec une quantité thérapeutiquement efficace d'un ou de plusieurs autres composés ou agents efficaces pour le traitement d'une maladie ou d'une infection hépatique ou pour la réduction du dépôt de graisse dans le foie ou de la fibrose du foie.

9. Composition pharmaceutique pour une utilisation dans un procédé de diminution du dépôt de graisse dans le foie selon la revendication 1, comprenant un antagoniste d'un récepteur de l'adénosine choisi dans le groupe constitué d'un antagoniste d'un récepteur A₁ et A_{2B} de l'adénosine et un support pharmaceutiquement acceptable.
